(19) Europäisches Patentamt / European Patent Office / Office européen des brevets

(11) **EP 3 473 215 B1**

(12) **EUROPEAN PATENT SPECIFICATION**

(45) Date of publication and mention
of the grant of the patent:
**31.07.2024 Bulletin 2024/31**

(21) Application number: **17813297.3**

(22) Date of filing: **13.06.2017**

(51) International Patent Classification (IPC):
**A61F 7/03** *(2006.01)* **A61F 7/00** *(2006.01)*

(52) Cooperative Patent Classification (CPC):
**A61F 7/00; A61F 7/034; A61F 7/03;**
A61F 2007/0006; A61F 2007/0017;
A61F 2007/0062

(86) International application number:
**PCT/JP2017/021782**

(87) International publication number:
**WO 2017/217401 (21.12.2017 Gazette 2017/51)**

(54) **HOT STEAM TOOL**

HEISSDAMPFINSTRUMENT

OUTIL À VAPEUR CHAUDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.06.2016 JP 2016120864**

(43) Date of publication of application:
**24.04.2019 Bulletin 2019/17**

(73) Proprietor: **Kao Corporation**
**Chuo-Ku**
**Tokyo 103-8210 (JP)**

(72) Inventors:
• **TSUCHIYA Shigemi**
**Tokyo 131-8501 (JP)**
• **SAITA Yasuto**
**Tokyo 131-8501 (JP)**
• **ODA Hideshi**
**Tokyo 131-8501 (JP)**

(74) Representative: **Hoffmann Eitle**
**Patent- und Rechtsanwälte PartmbB**
**Arabellastraße 30**
**81925 München (DE)**

(56) References cited:
WO-A1-2006/046658 JP-A- 2004 016 753
JP-A- 2006 102 145 JP-A- 2011 136 060
JP-A- 2011 136 060 JP-A- 2011 188 947

**Description**

TECHNICAL FIELD

The present invention relates to a steam heating tool.

BACKGROUND ART

It is known that a mask blocks a dust, a pollen, and the like in outside air by covering a mouth and a nose, and has preventive effects such as allergic rhinitis and a cold. In recent years, the function of the mask covering the mouth and the nose has diversified, and various masks have been developed. Among these, the effect due to steam is noted, and there is an increasing demand for a mask that can effectively supply steam to a throat and a nose.
For example, Patent Document 1 discloses a steam generation tool provided with a heating element generating steam in a mask body having a hollow preserved three-dimensional shape. In addition, Patent Document 2 discloses a humidifier for facial wearing provided with a sheet deformable following a shape of the face from the viewpoint of moisturizing the face by fitting the humidifier to the face. Patent Document 3 describes a steam generating tool that uses a composition to generate steam by an oxidation reaction of metallic powder containing 25-60 wt% of metallic powder, 25-60 wt% of water, salts, and a water absorptive polymer. A mass ratio of water absorptive polymer/water is 1/5 to 1/3 and metallic powder is adhered on the water absorptive polymer with absorbed water. Patent document 4 describes a nasal region heating implement for a mask that is arranged on the inner surface of the mask. The nasal region heating implement includes a heating element which generates moisture, and a storage body for housing the heating element. The storage body is formed to be swollen by the moisture generated along with the generation of the heat from the heating element.

RELATED DOCUMENT

PATENT DOCUMENT

[Patent Document 1] Japanese Unexamined Patent Publication No. 2004-73828
[Patent Document 2] Japanese Unexamined Patent Publication No. 2004-358110
[Patent Document 3] JP 2004 016753 A
[Patent Document 4] JP 2011 136060 A
[Patent Document 5] JP 2004 16753 A

SUMMARY OF THE INVENTION

According to the present invention, there is provided a steam heating tool as defined in appended claim 1 which includes a cover member that has a cover portion covering a mouth and a nose of a user, and a steam generator that is held on a surface of the cover portion on a side of the user, in which a space interposed between the cover portion and a face of the user is formed at a time of use, and the steam heating tool satisfies following formulae (1) to (3) .

$$300 \leq VG \leq 1,500 \quad (1)$$

$$40 \leq MV \leq 150 \quad (2)$$

$$5 \leq VG/MV \leq 25 \quad (3)$$

In the formulae (1) to (3), VG represents a cumulative steam generation amount [mg] released into the space for 10 minutes from a start of steam generation of the steam generator, and MV represents a volume [mL] of the space.
Further embodiments are described in the dependent claims. Methods of therapeutic use described in the following detailed description are illustrative only and not part of the present invention.

BRIEF DESCRIPTION OF THE DRAWINGS

The above objects and other objects, features and advantages will be more apparent by the preferred embodiments described below and the accompanying drawings attached thereto.

Fig. 1 is a perspective view schematically showing a steam heating mask according to a first embodiment.
Fig. 2 is a cross-sectional view of a mask in a use state of the steam heating mask according to the first embodiment.
Fig. 3 is a plan view of a portion of the steam heating mask according to the first embodiment when seen from a surface on a side of a user.
Fig. 4 is a cross-sectional view of a portion of the steam heating mask according to the first embodiment when seen from an upper surface (eye side of user).
Fig. 5 is a plan view of a modified example of the steam heating mask according to the first embodiment when seen from a surface on a side of a user.
Fig. 6 is a plan view of a modified example of the steam heating mask according to the first embodiment when seen from the surface on the side of the user.
Fig. 7 is a partial plan view when seen from a surface on a side of a user before using a steam generator to a steam heating mask according to a third embodiment.
Fig. 8 is a cross-sectional view of a portion of the steam heating mask according to the third embodiment when seen from an upper surface (eye side of user).
Fig. 9 is a cross-sectional view showing an example of a steam generator.
Fig. 10 is a schematic diagram showing an air permeation resistance measuring device.
Fig. 11 is a schematic diagram showing a device measuring the amount of steam generation.
Fig. 12 is a perspective view schematically showing a steam heating mask according to a second embodiment.
Fig. 13 is a cross-sectional view of the steam heating mask according to the second embodiment in a state of use.
Fig. 14 is a schematic diagram showing a method of measuring the temperature of a nose portion.

DESCRIPTION OF EMBODIMENTS

As a result of studies by the present inventors, in the technology disclosed in the above documents in the related art, it was found that there is room for improvement in the following points.

In the technology disclosed in Patent Document 1, since a volume of a hollow portion of the mask body was large, there was room for improvement in the balance between the amount of steam and the effect by steam, and the technology did not fully satisfy a high demand of a consumer. In addition, Patent Document 2 merely discloses a technology for fitting the humidifier to a face, and neither the amount of steam generation by the humidifier nor the effect by steam is described at all.

As a result of present intensive studies in order to respond to the high demand of the consumer and to efficiently obtain the effect by steam, the inventors were found that satisfying a specific condition in a steam heating tool was effective as a guide for solving the above problems. That is, it was found that it is possible to feel the improvement of feeling of steam, feeling of relaxation, and feeling of dryness of nose and throat by combining an indicator of a cumulative steam amount for 10 minutes from the start of steam generation of a steam generator with a space volume covering a nose and a mouth of a user and by highly controlling these balances, and thus the present invention was completed.

According to the present invention, it is possible to provide a steam heating tool which can obtain feeling of steam and feeling of relaxation, and improve feeling of dryness of the nose and throat.

Hereinafter, embodiments of the present invention will be described with reference to the drawings. In all the drawings, similar components are denoted by the same reference numerals, and description thereof will not be repeated as appropriate. In addition, "to" in the present specification represents "equal to or more" and "equal to or less" unless otherwise specified. In addition, the structures and elements described in each of the embodiments can be appropriately combined as long as the effects of the invention are not impaired.

In the present embodiment, a degree of air permeability of a sheet or the like can be measured as follows.

A degree of air permeability is a value measured by JIS P8117 (2009 revised edition) and is defined as a time for 100 ml of air to pass through an area of 6.42 cm$^2$ under a fixed pressure. Therefore, the fact that a numerical value of the degree of air permeability is high means that it takes time to pass air, that is, air permeability is low. Conversely, the fact that a numerical value of the degree of air permeability is low means that the air permeability is high. In this manner, a magnitude of the numerical value of the degree of air permeability and a high or low air permeability show an inverse relationship. The degree of air permeability can be measured by Oken type air permeability meter.

In the present specification, those having a degree of air permeability of equal to or more than 30,000 sec/100 ml are treated as "hardly air permeable", and those having a degree of air permeability of equal to or more than 80, 000 sec/100 ml are treated as "air impermeable".

(First Embodiment)

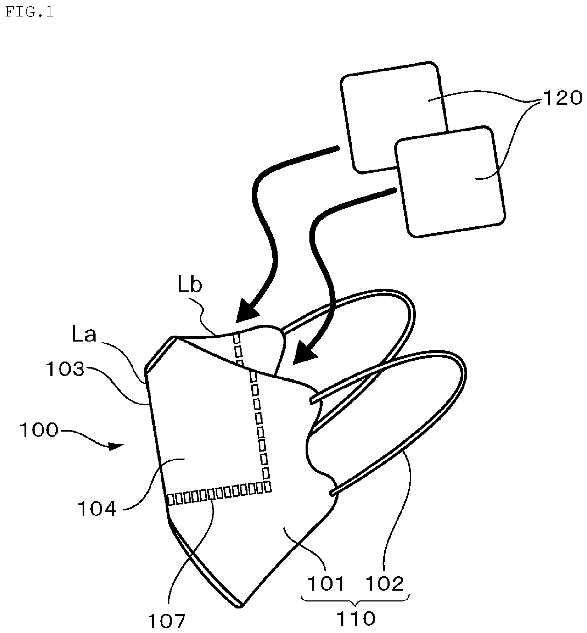

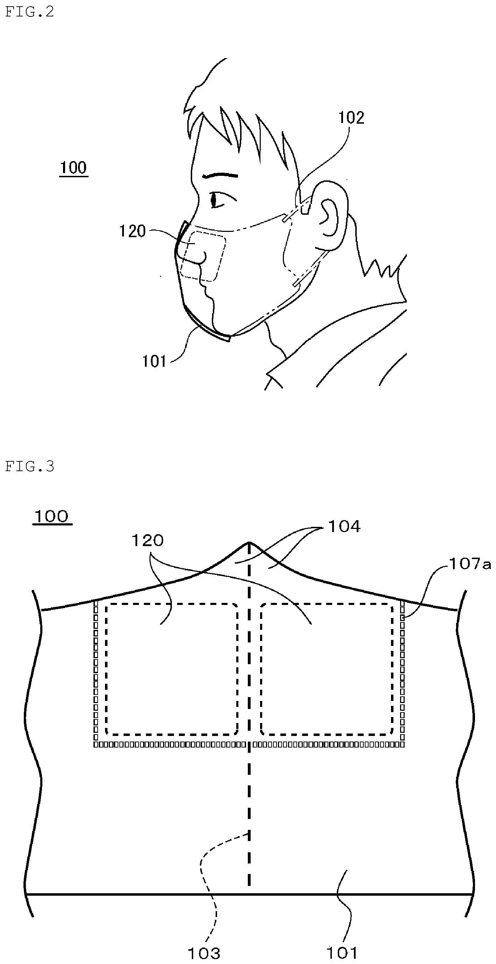

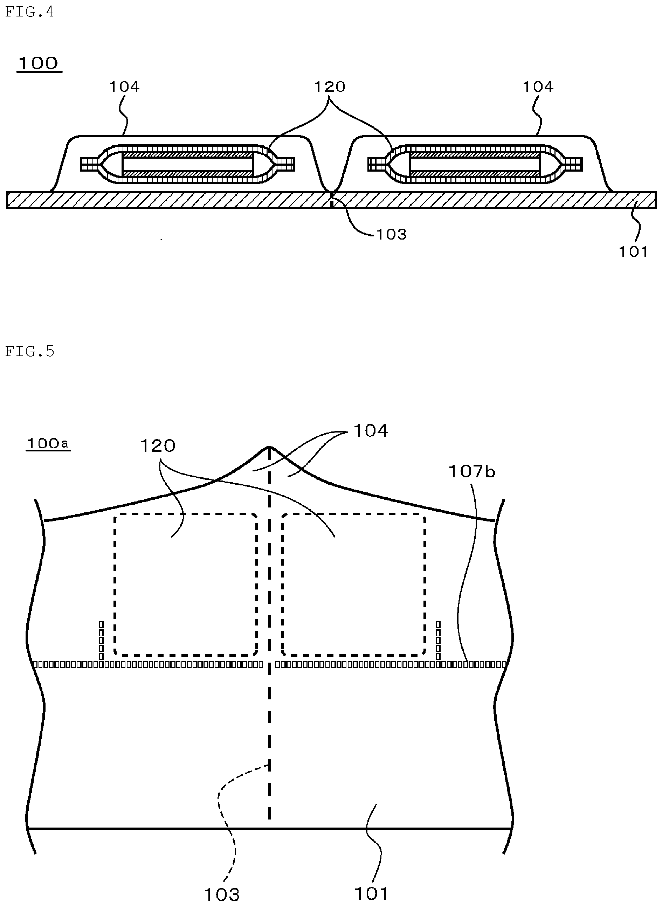

Fig. 1 is a perspective view showing an example of a steam heating mask 100. The steam heating mask 100 is provided with a mask 110 having a mask body portion 101, and a steam generator 120. Fig. 2 is a cross-sectional view of an example of the steam heating mask 100 in a use state. Fig. 3 is a plan view of a portion of the mask 110 according to the first embodiment when seen from a surface on a side of a user. Fig. 4 is a cross-sectional view of a portion of the mask according to the first embodiment when seen from an upper surface (eye side of user).

In the present embodiment, a steam heating mask set is described, in which the steam heating mask 100 are separated as the mask 110 and the steam generator 120, and the steam generator 120 can be taken in and out from a housing body 104, and housed and used in the housing body 104 at the time of use. However, the steam heating mask 100 may be a mask in which the steam generator 120 is previously sealed in the housing body 104 of the mask 110.

[Steam Heating Mask]

The steam heating mask 100 is provided with the mask 110 having the mask body portion 101 covering the mouth and the nose of the user, and the steam generator 120 held on a surface of the mask body portion 101 on a side of the user, in which a space interposed between the mask body portion 101 and a face of the user is formed at the time of use, and the steam heating tool satisfies following formulae (1) to (3).

$$300 \leq VG \leq 1,500 \qquad (1)$$

$$40 \leq MV \leq 150 \qquad (2)$$

$$5 \leq VG/MV \leq 25 \qquad (3)$$

In the formulae (1) to (3), VG represents a cumulative steam generation amount [mg] released into the space for 10 minutes from the start of steam generation of the steam generator 120, and MV represents a volume [mL] of the space.

VG represents the cumulative steam generation amount [mg] released into the space for 10 minutes from the start of steam generation of the steam generator 120. That is, after the steam heating mask 100 is worn, it is possible to more effectively apply the effect of steam by supplying an appropriate amount of steam in the first 10 minutes. In addition, by the effect of steam being impressed in the first 10 minutes, the effect will be sustained even afterwards, and the feeling of relaxation or feeling of moisture of the nose and throat is applied. VG is equal to or more than 300, and is preferably equal to or more than 400 from the viewpoint of applying more effect by steam.

MV represents the volume [mL] of the space, and is the volume of the space interposed between the mask body portion 101 and the face of the user when the steam heating mask 100 is used. For example, as shown in Fig. 2, when the steam heating mask 100 is properly worn, the space interposed between the mask body portion 101 and the face of the user is formed. That is, the space is a space interposed between the surface of the mask body portion 101 on the side of the user and the face of the user.

Here, the space interposed between the mask body portion 101 and the face of the user is not limited to a closed space formed by the mask body portion 101 being in contact with the face of the user, and may be a space having a slight gap between the mask body portion 101 and the face of the user. The gap is formed when the mask is worn in a normal manner and is of such a size that the effect of the steam generation by the steam generator 120 is not impaired. In addition, the volume of the space when there is a gap can be measured on the assumption that the space is formed along the shape of an edge portion of the mask body portion 101 in a gap forming portion.

The MV can be measured as follows. Using the human head model modeled using the average human head data (latest data at the time of application) made by Digital Human Technology Co., Ltd., it is possible to reproduce the use state of the steam heating mask 100, convert the use state into three-dimensional data, and measure the MV. In addition, as shown in Japanese Unexamined Patent Publication No. 2012-205926, substantially the same MV value can be obtained even by a method of disposing a liquid-impermeable film inside the mask, pouring water into this part, placing the face downwards so that it is in the same state as the mask is worn, and measuring the amount of remaining water after the face was taken off from the mask. Therefore, in a case where it is difficult to convert three-dimensional data depending on the shape of the steam heating mask, the MV may be measured by the method disclosed in Japanese Unexamined Patent Publication No. 2012-205926.

VG/MV is equal to or more than 5 and equal to or less than 25, and is preferably equal to or more than 10 and equal to or less than 15 from the viewpoint of balance of improvement of the feeling of steam, the feeling of relaxation, and the feeling of dryness.

[Measurement Method of Amount of Steam Generation]

The amount of steam generation can be measured as follows.

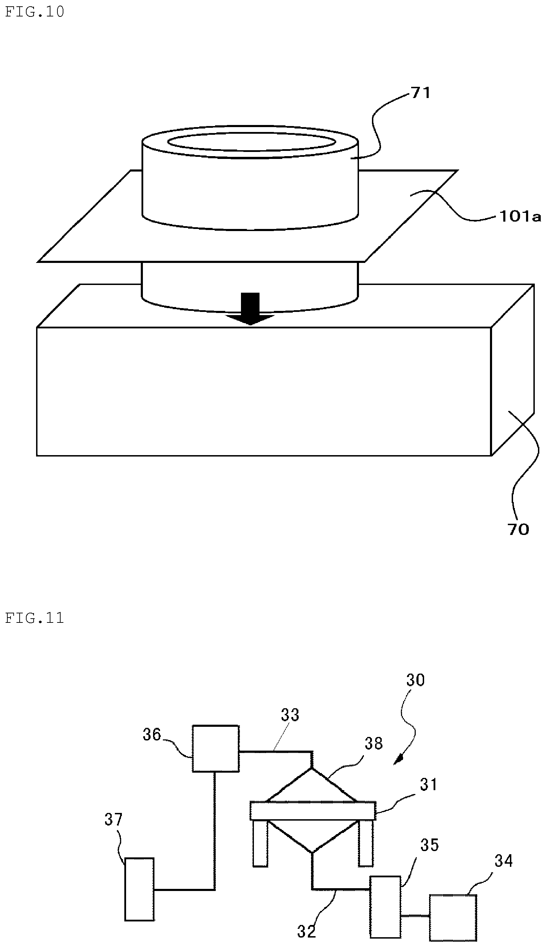

The amount of steam generation of the steam generator 120 is a numerical value measured using a device 30 shown in Fig. 11 as follows . The device 30 as shown in Fig. 11 is configured to include a measurement chamber (volume 4.2 L) 31 made of aluminum, an inflow path 32 allowing dehumidified air (humidity less than 2%, flow rate 2.1 L/min) to flow into a lower portion of the measurement chamber 31, an outflow path 33 through which air flows out from a upper portion of the measurement chamber 31, an inlet temperature and humidity meter 34 and an inlet flow meter 35 provided in the inflow path 32, an outlet temperature and humidity meter 36 and an outlet flow meter 37 provided in the outflow path 33, and a thermometer (thermistor) 38 provided in the measurement chamber 31. As the thermometer 38, one having a temperature resolution of approximately 0.01°C is used.

The measurement of the surface temperature of a surface located on a skin side of the steam generator 120 is measured by taking out the steam generator 120 from an oxygen blocking bag at a temperature of 30°C (30°C ± 1°C) in measurement environment, placing the surface located on the skin side of the steam generator 120 or the surface located on the skin side of the steam heating mask 100 facing upward, that is, placing a surface releasing the steam facing upward on the measurement chamber 31, and placing the thermometer 38 equipped with a metal ball (4.5 g) on the above-mentioned surface releasing the steam.

In addition, the dehumidified air is flown from the lower portion in this state, a difference between an absolute humidity before and after the air flows into the measurement chamber 31 from the temperature and the humidity measured by the inlet temperature and humidity meter 34 and the outlet temperature and humidity meter 36, and the amount of steam released by the steam generator 120 or the steam heating mask 100 is further calculated from the flow rate measured by the inlet flow meter 35 and the outlet flow meter 37. In the present specification, the amount of steam generation refers to the total amount of steam measured from the time when the steam generator 120 is taken out from the oxygen blocking bag as a starting point and until 10 minutes later.

The amount of steam generation of the steam heating mask 100 can be measured in the same manner as the above-described measurement method of the steam generator 120.

[Mask]

As shown in Figs. 1 and 2, the mask 110 includes the mask body portion 101 covering the nose and mouth of the user when worn, and a pair of ear hooking portions 102 provided at both left and right ends of the mask body portion 101. The mask body portion 101 has a folding line 103 at a vertical center portion. In the present embodiment, the folding line 103 is formed on a vertical center line bisecting the mask body portion 101 in a vertical direction. The folding line 103 has a protruded portion for allowing the mask body portion 101 to protrude along a nose portion at the time of use, and the upper portion and the lower portion are stuck each other. That is, the folding line 103 is a line extending from the upper end to the lower end of the mask body portion 101 and includes a region where the upper portion and the lower portion are stuck each other. The vertical center portion of the mask body portion 101 is a region having a width including a vertical center line and is a region in the vertical direction passing through the nose portion of the face when the mask 110 is properly used.

The mask body portion 101 is configured to be foldable, and folded in half by the folding line 103. The mask body portion 101 is mountain-folded along the folding line 103 and is in a state folded flat before use, and the mask body portion 101 is worn so as to be opened from the side opposite to the folding line 103 and an inner surface where the mask body portion 101 is folded is the surface on the side of the user at the time of use. The folding line 103 protrudes forward of the mask body portion 101 when the mask 110 is worn.

In the first embodiment, a space that covers the nose and mouth of the user is formed by being unfolded from the state folded in half by the folding line 103. Since the upper portion of the mask body portion 101 is brought into close contact with the shape of the nose by the folding line 103, the gap between the face of the user and the mask body portion 101 is unlikely to be formed, which is preferable in that the heating and moistening effect can be enhanced.

In the first embodiment, although the mask body portion 101 having the folding line 103 is described, a mask 110 having a flat shape without this folding line 103 may be used in accordance with the application or the like. As the mask 110 having the flat shape without the folding line 103, for example, there is a mask having a pleat portion in a lateral direction, described in the second embodiment mentioned later.

In Figs. 1 and 2, a maximum length (La) of the vertical center line of the mask body portion 101 and a length (Lb) of the upper side of the mask body portion 101 are shown.

The maximum length (La) of the vertical center line of the mask body portion 101 is a line perpendicular to the width direction (longitudinal direction) of the mask body portion 101. In addition, the line is a line passing through the center of the face of the user when wearing the mask 110 and bisecting the mask body portion 101. As shown in Fig. 1, in the first embodiment, the maximum length (La) of the vertical center line of the mask body portion 101 corresponds to the length of the folding line 103.

In the cross-sectional view of the mask body portion 101 on the vertical center line, the vertical center line is not limited to a straight line, and may be an arc shape, an uneven shape, or the like. In addition, the vertical center line may be a line actually existing in the mask body portion 101 or may be a virtual line.

In the first embodiment, the maximum length (La) of the vertical center line of the mask body portion 101 is preferably 12 to 20 cm. In this manner, a space of appropriate size is formed between the mask body portion 101 and the face of the user. As a result, the feeling of steam and the feeling of relaxation are easily obtained.

The length (Lb) of the upper side of the mask body portion 101 is a length from one end to the other end of the upper side of the mask body portion 101. In addition, the length (Lb) of the upper side of the mask body portion 101 is a length of a portion where the mask body portion 101 and the face of the user approach or are in contact with each other in a region located below the eyes of the user and extending from one cheek through the nose to the other cheek of the user when the mask 110 is worn.

In a case where the ear hooking portion 102 is formed at both end portions of the mask body portion 101 in the longitudinal direction, a joint between the mask body portion 101 and the ear hooking portion 102 serves as an end portion.

The upper side of the mask body portion 101 is not limited to a straight line, and may be an arc shape, a protruded portion, or a curve portion.

In the first embodiment, a ratio (La/Lb) of the maximum length (La) of the vertical center line of the mask body portion 101 to the sum of the length (Lb) of the upper side of the mask body portion 101 is preferably 0.6 to 1.7. In this manner, the mask 110 can come into close contact with the face of the user, and the space of an appropriate size is formed between the mask body portion 101 and the face of the user. As a result, the feeling of steam and the feeling of relaxation are easily obtained.

The mask body portion 101 has a sheet shape, and more specifically, the mask body portion 101 is formed of a single sheet.

Single sheet forming the mask body portion 101 may have a single structure (that is, one ply) or may have an integral structure (that is, multiply) by laminating a plurality of sheets. By using the plurality of sheets to impart separate functions to each sheet, it is preferable from the viewpoint that various functions can be imparted to the mask body portion 101. In a case where the plurality of sheets are used, the sheets may be in a laminated state in which the sheets are entirely bonded to each other, or in a state in which the sheets are separated from each other. In addition, the bonding of the sheets in the state where each of the sheets are separated from each other may seal the edges of each sheet along the shape of the mask body portion 101, or only a portion of the edge may be bonded by point sealing.

In the present embodiment, as shown in Fig. 4, an example in which the mask body portion 101 has a single structure will be described.

As the material of the mask body portion 101, one used in the technical field of the mask in the related art can be used, and there is no particular limitation on the type thereof as long as the material has a certain amount of air permeability. For example, a fiber sheet such as a nonwoven fabric or a gauze can be used, and it is preferable to use the nonwoven fabric from the viewpoints of ease of processing and economic efficiency. As the fiber material of the nonwoven fabric, fibers containing one or more selected from polyester such as polyethylene terephthalate (PET), polyolefins such as polyethylene (PE), polypropylene (PP), and ethylene propylene copolymer; rayon; cotton; and the like is preferable. In addition, as the nonwoven fabric, fabrics produced by an air-through method, a spunbond method, a needle punching method, a melt blown method, a card method, a heat fusion method, a water flow entangling method, a solvent adhesion method, or the like using fibers of one or more of the above materials can be used.

It is preferable that the mask body portion 101 has an appropriate air permeation resistance from the viewpoints of keeping steam generated from the steam generator 120 in the mask 110 and of easing breathing.

Specifically, the air permeation resistance of the mask body portion 101 is preferably equal to or more than 5 Pa, more preferably equal to or more than 20 Pa, and still more preferably equal to or more than 50 Pa. In addition, the air permeation resistance of the mask body portion 101 is preferably equal to or less than 200 Pa, more preferably equal to or less than 190 Pa, and still more preferably equal to or less than 180 Pa.

In addition, the air permeation resistance of the mask body portion 101 is preferably equal to or more than 5 Pa and equal to or less than 200 Pa, more preferably equal to or more than 20 Pa and equal to or less than 190 Pa, and still more preferably equal to or more than 50 Pa and equal to or less than 180 Pa. When the structure of the mask body portion 101 is multiplied, the air permeation resistance is an air permeation resistance which is measured in a state where all of the plural sheets are overlapped.

The air permeation resistance of the mask body portion 101 can be measured as follows.

As shown in Fig. 10, a sheet 101a cut into a size of 3.5 to 5 cm square from the sheet material of the mask body portion 101 is disposed on an upper portion of a main body 70 of an air permeation resistance evaluation device of mask tester MTS-2 (manufactured by Shibata Science Co., Ltd.), and the sheet 101a is fixed with a sheet fixing jig 71 so as not to leak. Measurement is performed for 10 seconds at a test area of 7 cm$^2$ (inside diameter 30 mm) and a test flow rate of 10 L/min, and the air permeation resistance is obtained from a differential pressure between an air inflow side (entrance side) and an air outflow side of the sheet 101a (exit side).

From the viewpoints of preventing the inside of the mask 110 from being seen through, and of balancing good heat retention, flexibility, thickness, sheet strength, a basis weight of the mask body portion 101 is preferably equal to or more than 5 g/m$^2$, more preferably equal to or more than 10 g/m$^2$, and still more preferably equal to or more than 30 g/m$^2$. In addition, the basis weight is preferably equal to or less than 200 g/m$^2$, more preferably equal to or less than 150 g/m$^2$, and still more preferably equal to or less than 120 g/m$^2$. In addition, the basis weight is preferably equal to or more than 5 g/m$^2$ and equal to or less than 200 g/m$^2$, equal to or more than 10 g/m$^2$ and more preferably equal to or less than 150 g/m$^2$, and still more preferably equal to or more than 30 g/m$^2$ and equal to or less than 120 g/m$^2$.

In the present embodiment, the steam heating mask 100 is provided with the steam generator 120. In the present embodiment, fixing means fixing the steam generator 120 to the mask body portion 101 is used, and the fixing means is fixedly used at the time of use. Specifically, as shown in Figs. 1 and 2, the mask body portion 101 is provided with a housing body 104 on the surface on the side of the user by a seal portion 107. The housing body 104 accommodates the steam generator 120 so that the steam generator 120 can be taken in and out freely. In this manner, after using the mask 110, the mask 110 can be used many times by replacing the steam generator 120 with a steam generator before use.

The steam generator 120 may be incorporated in the mask body portion 101 in advance.

As shown in Fig. 3, as a method of forming the housing body 104, the housing body 104 can be formed by the seal portion 107a that surrounds the outer periphery of the two steam generators 120 disposed in the lateral direction excluding the upper portion. Specifically, there are a method of overlapping the sheets forming the housing body 104 on the surface of the mask body portion 101 on the side of the user and sealing the seal portion 107a shown in Fig. 3 by heating fusion bonding or the like, and a method of overlapping the sheets forming the housing body 104 when preparing the mask body portion 101, sealing a region in the vicinity including the vertical center line of the mask body portion 101, and thereafter sealing the seal portion 107a shown in Fig. 3, and the like. In this manner, the housing body 104 in a pocket shape into which the steam generator 120 can be inserted from above the mask body portion 101 can be formed.

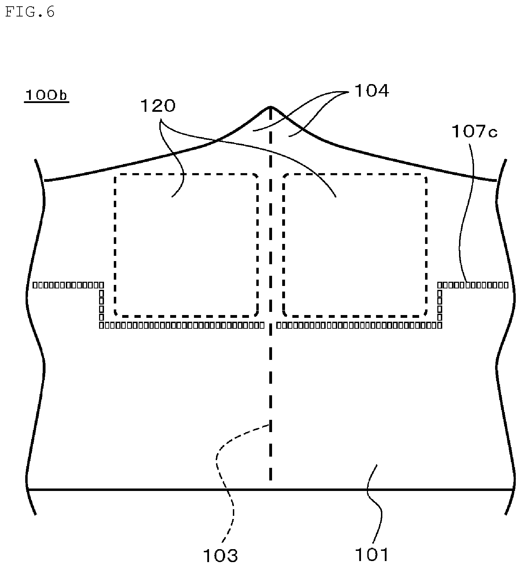

The method of forming the housing body 104 is not limited thereto. Figs. 5 and 6 are plan views of modified examples of the steam heating mask according to the first embodiment when seen from the surface on the side of the user. That is, as shown in Figs. 5 and 6, the housing body 104 may be formed by a sheet forming the mask body portion 101, and a seal portion 107b or a seal portion 107c to which the sheets forming the housing body 104 are fixed at a position where a portion of the lower side of the steam generator 120 is fixed. In Fig. 5, the seal portion 107b including a straight line which is a lateral direction of the central portion of the mask body portion 101 of the steam heating mask 100a and extending to be in contact with the lower side of the steam generator 120, and a straight line in the vertical direction extending in a portion of the lower portion of the side surface of the steam generator 120 on the side of the ear hooking portion 102 is shown. In Fig. 6, the seal portion 107c having a deformed linear shape along the shape of the lower portion of the steam generator 120 in a lateral direction slightly above the central portion of the mask body portion 101 of a steam heating mask 100b is shown.

In the present embodiment, it is preferable that the housing body 104 is fixed to the vicinity of the vertical center line of the mask body portion 101 and the vicinity of the upper end portion of the mask body portion 101 in view of the fact that the steam generator 120 can be fixed to the mask body portion 101 at a predetermined position, and it is easy to heat and moisten the space inside the mask 110 from the nose to the cheek of the user.

In the present embodiment, the housing body 104 has an opening portion so that the steam generator 120 can be taken in and out from the upper end portion or the ear hooking portion 102 side, and the other end portion is fixed to the mask body portion 101. The position of the opening portion is not particularly limited as long as the steam generator 120 does not be taken out outside the housing body 104 when the mask 110 is worn. In addition, the size of the housing body 104 may be any size as long as the position of the steam generator 120 can be fixed while accommodating the steam generator 120.

The housing body 104 can be made of the material having the air permeability and the same material as the mask body portion 101. From the viewpoint of effectively imparting the heating and moistening performance by the steam generator 120 to the inside of the mask 110 while preventing excessive heat generation, the air permeation resistance of the housing body 104 is preferably equal to or more than 1 Pa and equal to or less than 100 Pa, more preferably equal to or more than 1 Pa and equal to or less than 50 Pa, and still more preferably equal to or more than 1 Pa and equal to or less than 30 Pa.

The ear hooking portion 102 fixes the steam heating mask 100 to the face of the user. The ear hooking portion 102 is provided as a pair at the left and right end portions of the mask body portion 101 in the longitudinal direction (X direction).

In the present embodiment, as shown in Figs. 1 and 2, the ear hooking portion 102 is formed at the end of the mask body portion 101 with a cord-like material having elasticity such as a rubber string.

The ear hooking portion 102 may be the same material as the mask body portion 101 or may be a different material from the mask body portion 101.

The ear hooking portion 102 may be formed using a member having elasticity integrated with the mask body portion 101.

[Steam Generator]

The steam generator 120 is held at the surface of the mask body portion 101 on the side of the user. The steam generator 120 is preferably held in a region covering the nose of the user.

More specifically, from the viewpoint of creating a space surrounded by a face hollow between the nose portion and the cheek portion and the steam generator 120 on the side of the user of the mask body portion 101 when worn, and increasing the temperature and the absolute humidity inside the mask 110 without preventing from generating steam, as shown in Fig. 1, it is preferable to be attached symmetrically in the vicinity of the vertical center line of the mask body portion 101 and in the vicinity of the upper end of the mask body portion 101.

The vicinity of the vertical center line and the vicinity of the upper end portion of the mask body portion 101 are not limited to a case where these are in contact with the vertical center line and the upper end portion of the mask body portion 101, indicates regions around the folding line 103 and the upper end portion of the mask body portion 101, and are the regions where the steam generator 120 attached to the mask body portion 101 covers the nose portion of the user.

In addition, from the viewpoint of increasing the temperature and the absolute humidity inside the mask 110 without preventing from generating steam when the mask 110 is worn, the position of the steam generator 120 is such that the average of the shortest distances from the vertical center lines at both ends of the straight line in a case where the end portion of the steam generator 120 on the nose side is linear, and the shortest distance from the vertical center line of the curve in a case where the end portion of the steam generator 120 on the nose side is curved, are preferably equal to or less than 15 mm, more preferably equal to or less than 10 mm, and still more preferably equal to or less than 5 mm. In addition, from the same viewpoint, the position of the steam generator 120 is such that the shortest distance from the upper end portion of the mask body portion 101 on the upper end portion of the steam generator 120 is preferably equal to or less than 15 mm, more preferably equal to or less than 10 mm, and still more preferably equal to or less than 5 mm.

The steam generator 120 is preferably disposed so as to cover at least a portion of the nose portion of the user, and it is preferable that the steam generator 120 is disposed over the nose portion to the cheek portion from the viewpoint that a space surrounded by the hollow of the face between the nose portion and the cheek portion and the steam generator 120 is formed on the skin side of the mask body portion 101 when worn and of increasing the temperature and the absolute humidity inside the mask 110 without preventing from generating steam.

In addition, the planar shape of the steam generator 120 is not particularly limited, and may be circular, polygonal, or the like. From the viewpoints of producing efficiency, ease of handling, and heating and moistening effect, the planar shape is preferably a tetragon such as a rectangle and a substantially square, and more preferably a substantially square from the viewpoint of ease of handling. In addition, in a case where the end portion of the steam generator 120 on the nose side is linear, it is preferable that a portion of the vertical center line of the mask body portion 101 in contact with the steam generator 120 is linear.

In a case where the steam generator 120 can be attached and detached from the mask 110, the steam generator 120 is contained in the oxygen blocking bag before use.

The oxygen blocking bag has an oxygen blocking property as a whole, so that the steam generator 120 does not come into contact with oxygen in the air. As the oxygen blocking property of the oxygen blocking bag, for example, a material having an oxygen permeability coefficient (ASTM D 3985) of equal to or less than 10 $cm^3 \cdot mm/(m^2 \cdot day \cdot MPa)$, and particularly equal to or less than 2 $cm^3 \cdot mm/(m^2 \cdot day \cdot MPa)$ is preferable. Specifically, there is a film such as ethylene-vinyl alcohol copolymer and polyacrylonitrile, or a film obtained by depositing ceramic, aluminum or the like on such a film, and the like.

In addition, in a case where the steam generator 120 is sealed in the mask 110, the entire mask 110 may be sealed in the oxygen blocking bag to avoid contact between the steam generator 120 and oxygen in the air.

[Steam Generating Portion]

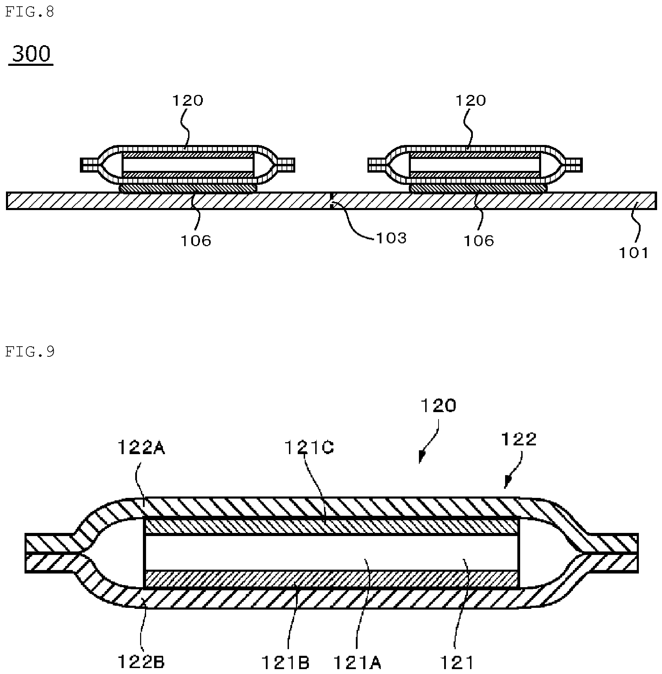

As shown in Fig. 9, the steam generator 120 internally accommodates a steam generating portion 121. In the present embodiment, the steam generator 120 has the steam generating portion 121 and a bag 122 accommodating the steam generating portion 121. The bag 122 is provided with a first sheet 122A on a surface on the side of the user (skin side) and a second sheet 122B on a surface opposite to the surface on the side of the user (skin side).

The steam generator 120 generates heat and generates steam by reacting with oxygen in the air. In this manner, warmed steam can be supplied.

The steam generating portion 121 may take various forms. The steam generating portion 121 may be, for example, a sheet form such as a powder mixture or a papermaking sheet, or a coated sheet coated with a dispersion liquid or the like on a substrate.

The steam generating portion 121 includes a form in which an oxidizable metal, a water absorbing agent, water, an electrolyte and, if necessary, a reaction accelerator and the like are contained.

When the steam generating portion 121 comes into contact with air, an oxidation reaction of the oxidizable metal contained therein occurs, and heat is generated. Due to this heat, the water contained in the steam generating portion 121 is heated and becomes steam of a predetermined temperature, and is released to the outside through the bag 122. Steam is released to the outside from the air permeability portion of the bag 122. In this manner, steam can be obtained in a simple way.

The oxidizable metal is a metal that generates heat of oxidation reaction, and examples thereof include one or more powders or fibers selected from iron, aluminum, zinc, manganese, magnesium, and calcium. Among these, iron powder is preferable from the viewpoints of handling property, safety, production cost, storability, and stability. Examples of the iron powder include one or more selected from reduced iron powder and atomized iron powder.

In a case where the oxidizable metal is powder, from the viewpoint that the oxidation reaction is efficiently performed, the average particle diameter thereof is preferably equal to or more than 0.1 pm, more preferably equal to or more than 10 pm, and still more preferably equal to or more than 20 $\mu$m. From the same viewpoint, the average particle diameter is preferably equal to or less than 300 pm, more preferably equal to or less than 200 pm, and still more preferably equal to or less than 150 $\mu$m.

Furthermore, from the viewpoint of improving the coatability, the average particle diameter is preferably equal to or more than 10 $\mu$m and equal to or less than 200 $\mu$m, and the average particle diameter is more preferably equal to or more than 20 $\mu$m and equal to or less than 150 $\mu$m.

In addition, from the viewpoint of improving the fixability of fibrous materials and the like to a water retention material and improving control of the reaction, it is also preferable to use one containing equal to or more than 50% by mass of particles having a particle size of 0.1 to 150 $\mu$m.

The particle diameter of the oxidizable metal refers to a maximum length in the form of a powder, and the particle diameter is measured by sieve classification, a dynamic light scattering method, a laser diffraction and scattering method or the like.

The content of the oxidizable metal in the steam generating portion 121 is preferably equal to or more than 100 g/m$^2$, and more preferably equal to or more than 200 g/m$^2$ in terms of basis weight. In addition, the content of the oxidizable metal in the steam generating portion 121 is preferably equal to or less than 3, 000 g/m$^2$, and more preferably equal to or less than 1,600 g/m$^2$ in terms of basis weight.

In addition, the content is preferably equal to or more than 100 g/m$^2$ and equal to or less than 3,000 g/m$^2$, and more preferably equal to or more than 200 g/m$^2$ and equal to or less than 1,600 g/m$^2$ in basis weight. In this manner, the heat generation temperature of the steam generator 120 can be raised to a desired temperature.

Here, the content of the oxidizable metal can be obtained by ash test according to JIS P 8128 or thermogravimetric instrument. In addition, the content can be quantified by a vibration sample type magnetization measurement test or the like by utilizing the property that magnetization occurs when an external magnetic field is applied.

The type of the water absorbing agent is not particularly limited as long as the water absorbing agent can hold water, and examples thereof include one or more selected from a carbon component, a fibrous material, a water absorbing polymer, and a water absorbing powder. As the water absorbing agent, an appropriate one is used according to the form of the steam generating portion 121.

As the carbon component, one having water retention ability, oxygen supply ability, and catalytic ability can be used. For example, one or more selected from activated carbon, acetylene black, and graphite can be used. Among these, activated carbon is preferable, and one or more fine powdery materials or small granular materials selected from coconut shell carbon, wood charcoal, and peat coal are more preferable. Among these, from the viewpoint of obtaining a favorable heating and moistening effect, wood charcoal is still more preferable.

The average particle diameter of the water absorbing agent is preferably equal to or more than 10 pm, and more preferably equal to or more than 12 $\mu$m. In addition, the average particle diameter of the water absorbing agent is preferably equal to or less than 200 pm, and more preferably equal to or less than 100 $\mu$m.

In addition, the average particle diameter of the water absorbing agent is preferably equal to or more than 10 $\mu$m and equal to or less than 200 $\mu$m, and the average particle diameter is more preferably equal to or more than 12 $\mu$m and equal to or less than 100 $\mu$m.

The average particle diameter of the water absorbing agent refers to the maximum length in the form of powder and is measured by dynamic light scattering method, laser diffraction method, and the like. It is preferable to use a powdery form of the carbon component, and it is also possible to use a form other than the powdery form, for example, a fibrous form.

As the fibrous material, natural or synthetic fibrous materials can be used without particular limitation.

Examples of natural fibrous materials include vegetable fibers such as cotton, kapok, wood pulp, non-wood pulp, peanut protein fiber, corn protein fiber, soy protein fiber, mannan fiber, rubber fiber, hemp, manila hemp, sisal hemp, New Zealand hemp, luobuma hemp, palm, tampa, and wheat straw. In addition, examples thereof include animal fibers such as wool, goat hair, mohair, cashmere, alpaca, angora, camel, vikuna, silk, plumes, down, feather, algin fiber, chitin fiber, and casein fiber. Furthermore, examples thereof include mineral fibers such as asbestos.

On the other hand, examples of synthetic fibrous materials include semisynthetic fibers such as rayon, viscose rayon, cupra, acetate, triacetate, oxidized acetate, prommix, chlorinated rubber, and hydrochloric acid rubber. Examples thereof include polyester such as polyethylene terephthalate, and synthetic polymeric fibers such as polyacrylonitrile, acrylic, polyethylene, polypropylene, polystyrene, and polyurethane, in addition to nylon, aramid, polyvinyl alcohol, polyvinyl chloride, and polyvinylidene chloride. Furthermore, metal fibers, carbon fibers, glass fibers, and the like can also be used. These fibers can be used alone or in mixture . Among these, wood pulp, cotton, polyethylene fiber, polyester fiber are preferably used from the viewpoint of fixability with an oxidizable metal and a reaction promoter, flexibility of the steam generating portion 121, oxygen permeability, maintenance function of sheet form, production cost, and the like. In addition, wood pulp and cotton have a function to support and immobilize solid materials such as iron powder.

Examples of the water absorbing polymer include a hydrophilic polymer having a crosslinked structure capable of absorbing and holding a liquid equal to or more than 20 times of its own weight, and the like.

Examples of the water absorbing powder includes one or more selected from vermiculite, calcium silicate, sawdust, alumina, silica gel, and pulp powder.

In a case where the steam generating portion 121 is in the form of a sheet, it is preferable to use a fibrous material as the water absorbing agent. The reason for this is that the fibrous material has both the function as the water retention material and the function of maintaining the sheet form by the steam generating portion 121. As a result, uneven distribution of the oxidizable metal is unlikely to occur, and the steam generating portion 121 has a uniform heat generation temperature distribution.

In a case where the steam generating portion 121 is a mixture formed of powders, it is preferable to use a superabsorbent polymer, vermiculite, calcium silicate, silica gel, silica type porous material, alumina, wood powder, or the like as the water absorbing agent.

The content of the water absorbing agent is preferably equal to or more than 0.3 parts by mass, more preferably equal to or more than 1 part by mass, and still more preferably equal to or more than 3 parts by mass based on 100 parts by mass of the oxidizable metal. In addition, the content of the water absorbing agent is preferably equal to or less than 100 parts by mass, more preferably equal to or less than 80 part by mass, and still more preferably equal to or less than 60 parts by mass based on 100 parts by mass of the oxidizable metal.

In addition, the content of the water absorbing agent is preferably equal to or more than 0.3 parts by mass and equal to or less than 100 parts by mass, more preferably equal to or more than 1 part by mass and equal to or less than 80 part by mass, and still more preferably equal to or more than 3 parts by mass and equal to or less than 60 parts by mass based on 100 parts by mass of the oxidizable metal. In this manner, moisture necessary for sustaining the oxidation reaction can be accumulated in the obtained steam generator 120. In addition, oxygen supply to the steam generating portion 121 is sufficiently obtained to obtain the steam generator 120 having high heat generation efficiency. In addition, since the heat capacity of the steam generator 120 can be prevented to a small value with respect to the obtained heating value, an increase in the heat generation temperature is increased, a desired temperature rise is obtained, and a heat generation reaction can be promoted.

The content of the water absorbing agent is preferably equal to or more than 4 $g/m^2$ and equal to or less than 290 $g/m^2$, and more preferably equal to or more than 7 $g/m^2$ and equal to or less than 160 $g/m^2$ in terms of basis weight. In this manner, it is possible to reduce the thickness of the steam generating portion 121, so that the steam generating portion 121 is not bulky as a product and has flexibility. For example, the thickness of the steam generating portion 121 can be set to equal to or more than 0.1 mm and equal to or less than 2 mm.

Examples of the electrolyte include sulfates, carbonates, chlorides or hydroxides of alkali metals, alkaline earth metals or transition metals. Among these, chlorides of alkali metals, alkaline earth metals or transition metals are preferably used from the viewpoint of excellent conductivity, chemical stability and production cost, and in particular, sodium chloride, potassium chloride, calcium chloride, magnesium chloride, ferrous chloride, ferric chloride are preferably used.

The steam generating portion 121 contains water. The water may be derived from an aqueous electrolyte solution (for example, an aqueous solution of an alkali metal, an alkaline earth metal or the like), or may be one which is added to the steam generating portion 121 with water alone, and is not particularly limited.

The moisture content in the steam generating portion 121 is preferably equal to or more than 35 parts by mass and equal to or less than 200 parts by mass based on 100 parts by mass of the oxidizable metal. By setting the moisture content of the steam generating portion 121 to equal to or less than 200 parts by mass based on 100 parts by mass of the oxidizable metal, the steam generating portion 121 generates heat well and the rise of the heat generation temperature is faster (temperature rise time is faster) . In addition, by setting the moisture content of the steam generating portion 121 to equal to or more than 35 parts by mass based on 100 parts by mass of the oxidizable metal, it is possible to ensure the moisture content necessary for the heat generation reaction in the steam generating portion 121 and to maintain the heat generation reaction in the steam generating portion 121 satisfactorily.

In this manner, by setting the moisture content of the steam generating portion 121 to equal to or more than 35 parts by mass and equal to or less than 200 parts by mass based on 100 parts by mass of the oxidizable metal, it is possible to obtain the steam generating portion 121 in a satisfactory heat generation state. That is, the moisture content of the steam generating portion 121 affects the heat generation rate. When the moisture content is equal to or more than 35 parts by mass and equal to or less than 200 parts by mass based on 100 parts by mass of the oxidizable metal, heat is generated well, the rise of the heat generation temperature is fast, and the heat generation temperature is maintained.

From the same viewpoint, the moisture content in the steam generating portion 121 is more preferably 40 parts by mass or more, and still more preferably 50 parts by mass or more based on 100 parts by mass of the oxidizable metal. In addition, the moisture content in the steam generating portion 121 is preferably equal to or less than 200 parts by mass, more preferably equal to or less than 150 parts by mass, still more preferably equal to or less than 100 parts by mass, and even more preferably equal to or less than 80 parts by mass based on 100 parts by mass of the oxidizable metal.

In addition, the moisture content in the steam generating portion 121 is more preferably equal to or more than 40 parts by mass and equal to or less than 150 parts by mass, still more preferably equal to or more than 50 parts by mass and equal to or less than 100 parts by mass, and even more preferably equal to or more than 50 parts by mass and equal to or less than 80 parts by mass based on 100 parts by mass of the oxidizable metal.

In addition to each of the above-described components, the steam generating portion 121 may contain a thickener, a surfactant, a medicine, a flocculant, a coloring agent, a paper strengthening agent, a pH adjusting agent (for example, tripotassium phosphate), a bulking agent, and the like.

As the thickener, materials that absorb moisture to increase the consistency or thixotropic properties can be used. For example, it is possible to use one or more selected from a polysaccharide type thickener such as alginate such as sodium alginate, gum arabic, gum tragacanth, locust bean gum, guar gum, carrageenan, agar, xanthan gum; a starch type thickener such as dextrin, pregelatinized starch, and processing starches; a cellulose derivative type thickener such as carboxymethyl cellulose, ethyl cellulose acetate, hydroxyethyl cellulose, hydroxymethyl cellulose or hydroxypropyl cellulose; a thickener such as polyvinyl alcohol (PVA) ; a metal soap type thickener such as stearate; a mineral type thickener such as bentonite, and the like. Among these, a polysaccharide type thickener is preferable, and xanthan gum is preferable from the viewpoint of keeping the moisture content in the steam generating portion 121 constant.

In a case where the steam generating portion 121 is a coated sheet, the content of the thickener is preferably equal to or more than 0.1 parts by mass, and more preferably equal to or more than 0.2 parts by mass based on 100 parts by mass of the oxidizable metal from the viewpoint of ease of coating. In addition, the content of the thickener is preferably equal to or less than 5 parts by mass, and more preferably equal to or less than 4 parts by mass based on 100 parts by mass of the oxidizable metal. The content of the thickener is preferably equal to or more than 0.1 parts by mass and equal to or less than 5 parts by mass, and more preferably equal to or more than 0.2 parts by mass and equal to or less than 4 parts by mass based on 100 parts by mass of the oxidizable metal.

In addition, in a case where the steam generating portion 121 is in the form of a sheet, it is preferable that multiple holes and/or incisions are formed. As a result, even if the sheet-like steam generating portion 121 is thin, high heat generation characteristics can be sufficiently obtained and desired steam release characteristics can be obtained. It is preferable that the area of the hole is 0.01 to 10 mm$^2$, and particularly 0.1 to 8 mm$^2$, since sufficient heat generation characteristics can be obtained. For the same reason, it is preferable that the holes are formed in the sheet-like steam generating portion 121 at 0.1 to 20 holes/cm$^2$, and particularly 1 to 15 holes/cm$^2$. The shape of the hole may be, for example, a circle, a rectangle, a polygon, an ellipse, an oval, or a combination of two or more of these. On the other hand, in a case of forming incisions, the length thereof is preferably 1 to 50 mm, and particularly preferably 5 to 30 mm.

The steam generating portion 121 is accommodated in the bag 122 provided with a first sheet 122A and a second sheet 122B of the steam generator 120. That is, the steam generator 120 is configured to include the first sheet 122A and the second sheet 122B. Preferably, by sealingly bonding the peripheral portions of the first sheet 122A and the second sheet 122B to each other, the bag 122 are formed. A region other than the peripheral portions of the first sheet 122A and the second sheet 122B is a non-bonded region, and the steam generating portion 121 is disposed in the non-bonded region.

In the present embodiment, the first sheet 122A is disposed on the surface of the steam generating portion 121 on the side of the user, and the second sheet 122B is disposed on the surface opposite to the surface of the steam generating portion 121 on the side of the user. Details of each degree of air permeability will be described below.

The degree of air permeability of the first sheet 122A is preferably equal to or less than 7,000 sec/100 ml. From the viewpoint of maintaining the space surrounded by the hollow of the face between the nose portion and the cheek portion, and the steam generator 120 to ensure air permeability and to easily release a large amount of steam from the steam generator 120 to the outside of the bag 122, the degree of air permeability of the first sheet 122A is more preferably equal to or less than 5,000 sec/100 ml, still more preferably equal to or less than 2,500 sec/100 ml, even more preferably equal to or less than 1,000 sec/100 ml, and further more preferable in the order of equal to or less than 600 sec/100 ml, equal to or less than 10 sec/100 ml, equal to or less than 5 sec/100 ml, and 0 second/100 ml.

As the first sheet 122A having such degree of air permeability, for example, it is preferable to use a synthetic resin porous sheet having moisture permeability, and not having water permeability. Specifically, a film obtained by stretching polyethylene or polypropylene containing calcium carbonate or the like can be used. In a case where such a porous sheet is used, various fiber sheets including one or more of nonwoven fabrics selected from needle-punched nonwoven fabric, air-through nonwoven fabric, and spunbonded nonwoven fabric may be laminated on the outer surface of the porous sheet to enhance the texture of the first sheet 122A.

In addition, when the above degree of air permeability is satisfied, the first sheet 122A may be an air impermeable sheet of which a portion does not have air permeability.

A portion of the second sheet 122B may be an air permeable sheet having air permeability or an air impermeable sheet not having air permeability, and a sheet having low air permeability is adopted as a whole. Specifically, the degree of air permeability of the second sheet 122B is preferably greater than 8,000 sec/100 ml, and from the viewpoint that the inside of the mask body portion 101 can be stably heated and humidified effectively, it is more preferable to be the air impermeable sheet.

When the above degree of air permeability is satisfied, according to the application, the second sheet 122B can also have various fiber sheets including one or more of nonwoven fabrics selected from needle-punched nonwoven fabric, air-through nonwoven fabric, and spunbonded nonwoven fabric laminated on a film made of one or multiple layers of synthetic resin or an outer surface of the film made of the one or multiple layers of synthetic resin to enhance the texture of the second sheet 122B. Specifically, a two-layer film made of a polyethylene film and a polyethylene terephthalate film, a laminate film made of a polyethylene film and a nonwoven fabric, a laminate film made of a polyethylene film and a pulp sheet, and the like are used, and a laminate film made of a polyethylene film and a pulp sheet is even more preferable.

The second sheet 122B may use the same material as the first sheet 122A or may use a different material as long as the second sheet 122B satisfies the above-described degree of air permeability value.

In addition, the degree of air permeability of the second sheet 122B is more preferably equal to or more than 10,000 sec/100 ml, still more preferably equal to or more than 30,000 sec/100 ml, and even more preferably equal to or more than 80,000 sec/100 ml. By setting the air permeability of the second sheet 122B in this manner, it is possible to efficiently release the steam generated in the steam generating portion 121 from the first sheet 122A side and prevent expansion of the steam generator 120.

In particular, from the viewpoint of making the oxidation reaction of the oxidizable metal favorable and easily generating a large amount of steam from the first sheet 122A side, it is even more preferable that the degree of air permeability of the first sheet 122A is equal to or less than 2,500 sec/100 ml, and the degree of air permeability of the second sheet 122B is equal to or more than 80,000 sec/100 ml.

In this case, an air impermeable or hardly air permeable sheet, and more preferably the air impermeable sheet is disposed on the surface of the steam generating portion 121 on the side opposite to the surface located on the side of the user, that is, between the steam generating portion 121 and the outermost layer on the side opposite to the user of the steam generator 120. In this manner, it is possible to prevent the steam generated by the steam generating portion 121 from leaking to the outside of the mask 110 and apply the steam to the inside of the mask 110, that is, on the side of the user.

An example of a method for producing the steam generating portion 121 will be described below.

In a case where the steam generating portion 121 is in the form of a sheet, for example, the wet paper-making method described in Japanese Unexamined Patent Publication No. 2003-102761 according to the prior application of the present applicant or an extrusion method using a die coater can be used. In this case, first, a formed sheet including an oxidizable metal, a water absorbing agent, and a reaction accelerator is formed by a wet paper-making method, and an aqueous electrolytic solution is added to the formed sheet to obtain the steam generating portion 121 having a sheet shape. The obtained steam generating portion 121 having a sheet shape may be used as one piece, or a plurality of sheets may be overlapped and used. Alternatively, one steam generating portion 121 may be folded, and a plurality of folded steam generating portions 121 may be overlapped and used.

In a case where the steam generating portion 121 is made of powder, by mixing the constituent materials uniformly, the steam generating portion 121 of the powder is obtained. More specifically, first, a water absorbing agent such as a highly absorbent polymer and an oxidizable metal are uniformly mixed, and an electrolytic aqueous solution is added thereto to adhere the oxidizable metal to the surface of the water absorbing agent. Thereafter, the steam generating portion 121 can be prepared by adding a reaction accelerator or the like which is the remaining material. By preparing the steam generating portion 121 in this manner, the rise time of the oxidation reaction becomes earlier and an evaporation amount of steam per unit time becomes easily maximum.

In addition, in a case where the steam generating portion 121 is formed of a coated sheet, for example, the steam generating portion 121 may be one obtained by coating an aqueous dispersion of heat generation powder to a water retention sheet and cutting a continuous long object of heat generation material provided with a heat generation layer and a water retention sheet into an any size, according to the method described in Japanese Unexamined Patent Publication No. 2013-146554 according to the prior application of the present applicant. The steam generating portion 121 may be one sheet or may be accommodated in a multilayer state where plural sheets are stacked.

Here, the configuration of the steam generator 120 in the case where the steam generating portion 121 is formed of the coated sheet will be described below.

As shown in Fig. 9, the steam generating portion 121 has a steam generation layer 121A between a base material layer 121B and a water retention sheet 121C. The steam generation layer 121A and the water retention sheet 121C are in direct contact with each other. The steam generator 120 is equipped with the steam generating portion 121 in the bag 122 having the first sheet 122A and the second sheet 122B so that the water retention sheet 121C side, that is, the first sheet 122A side is positioned on the skin side of the user and the base material layer 121B is disposed on the side of the second sheet 122B. As a result of this, steam from the steam generating portion 121 can be efficiently discharged from the first sheet 122A.

The steam generation layer 121A may be provided on the one surface of the water retention sheet 121C or may be provided in a form interposed between the water retention sheet 121C and the base material layer 121B. Fig. 9 shows an example in which the steam generation layer 121A is provided so as to be interposed between the water retention sheet 121C and the base material layer 121B.

The water retention sheet 121C contains water. The content of water may be, for example, equal to or more than 10% by mass and equal to or less than 45% by mass of a maximum amount of water absorption of the water retention sheet 121C.

The maximum amount of water absorption of the water retention sheet 121C can be calculated as follows.

After measuring the weight ($W_0$) of the water retention sheet 121C cut to a size of 25 cm$^2$, the sheet is immersed in a sodium chloride aqueous solution of 5% by mass for 5 minutes. The sheet is removed with tweezers, hung in the air for 1 minute, and the dripping moisture that cannot be held is dropped. Thereafter, the mass ($W_1$) is measured and the maximum amount of water absorption ($W_{max}$) is calculated from the following equation.

$$W_{max} = W_1 - W_0$$

In addition, the moisture content contained in the water retention sheet 121C is preferably 50 to 350 g/m$^2$, and more preferably 180 to 260 g/m$^2$, in terms of basis weight. Since the moisture content contained in the water retention sheet 121C is a steam generation source, a preferable steam generation amount can be ensured by setting the moisture content contained in the water retention sheet 121C to preferably equal to or more than 50 g/m$^2$ in terms of basis weight. In addition, the water retention sheet 121C generates the air permeation resistance (air permeability decreases due to water absorption swelling as compared with drying) due to water absorption. Therefore, since steam is easily released from the water retention sheet 121C and sufficient air permeability to the steam generation layer 121A is ensured by setting the moisture content to preferably equal to or less than 350 g/m$^2$ in terms of basis weight, the steam generator 120 having sufficient oxygen supply and high heat generation efficiency can be obtained.

In addition, the degree of air permeability of the water retention sheet 121C is preferably equal to or less than 500 sec/100 ml in the state of containing moisture, and the degree of air permeability is more preferably equal to or less than 300 sec/100 ml, and still more preferably equal to or less than 50 sec/100 ml in consideration of the air permeability and ease of passing steam.

The lower limit value of the degree of air permeability in a state containing moisture (that is, moisture content is equal to or more than 15% by mass and equal to or less than 30% by mass of the maximum amount of water absorption of the water retention sheet 121C) is, for example, 1 sec/100 ml.

Here, as the water retention sheet 121C, a sheet material which can absorb and hold moisture and has flexibility is used. Examples of such a material include paper made from fiber, a fiber sheet such as nonwoven fabric, woven fabric, and knitted fabric. In addition, porous body such as sponge is included. Examples of the above fibers include a fiber based on a natural fiber such as a vegetable fiber and an animal fiber, and a fiber based on a chemical fiber. Examples of the vegetable fiber include one or more selected from cotton, kapok, wood pulp, non-wood pulp, peanut protein fiber, corn protein fiber, soy protein fiber, mannan fiber, rubber fiber, hemp, manila hemp, sisal hemp, New Zealand hemp, luobuma hemp, palm, tampa, and wheat straw. Examples of the animal fiber include one or more selected from wool, goat hair, mohair, cashmere, alpaca, angora, camel, vikuna, silk, plumes, down, feather, algin fiber, chitin fiber, and casein fiber. As the chemical fiber, one or more selected from rayon, acetate, and cellulose, for example, can be used.

Among these, as the water retention sheet 121C, a sheet containing a fiber material made of the above-described fibers and a water absorbing polymer is preferable.

It is preferable to use a hydrogel material capable of absorbing and holding a liquid equal to or more than 20 times of its own weight and capable of gelling as the water absorbing polymer because the water content contained in the water retention sheet 121C can be maintained at 15% to 30% by mass of the maximum amount of water absorption of the water retention sheet 121C.

The shape of the particles of the water absorbing polymer may be spherical, lumpy, grape-like, fibrous, and the like.

In addition, the particle diameter of the water absorbing polymer particles is preferably equal to or more than 1 pm, and more preferably equal to or more than 10 $\mu$m, from the viewpoint of ease of handling during production. In addition, the particle diameter of the water absorbing polymer particles is preferably equal to or less than 1,000 pm, and more preferably equal to or less than 500 $\mu$m, from the viewpoint of water absorption speed.

In addition, the particle diameter of the water absorbing polymer particles is preferably equal to or more than 1 $\mu$m and equal to or less than 1,000 pm, and more preferably equal to or more than 10 $\mu$m and equal to or less than 500 $\mu$m.

The particle diameter of the water absorbing polymer particles is measured by a dynamic light scattering method, a laser diffraction method, or the like.

Specific examples of the water absorbing polymer include one or more selected from starch, crosslinked carboxyl methylated cellulose, a polyacrylic acid, salt thereof, and polyacrylate graft polymer such as a polymer or copolymer of acrylic acid or an alkali metal salt of acrylic acid. Among these, it is preferable to use a polyacrylic acid, salt thereof, and polyacrylate graft polymer such as a polymer or copolymer of acrylic acid or an alkali metal salt of acrylic acid.

The base material layer 121B is provided on the surface of the steam generation layer 121A on the side opposite to the water retention sheet 121C. The base material layer 121B is in direct contact with the steam generation layer 121A and covers the steam generation layer 121A. The base material layer 121B is preferably an air impermeable or hardly air permeable sheet, and for example, a resin sheet is preferably used. By forming the air impermeable or hardly air permeable sheet (it is equal to or more than 50,000 sec/100 ml, and it is preferably equal to or more than 80,000 sec/100 ml), not only the steam can be more reliably released from the water retention sheet 121C side, but also vaporization heat can be prevented from being deprived from the base material layer 121B side.

Examples of the base material layer 121B include a synthetic resin film, a polyethylene film, a polyethylene terephthalate film, and the like.

In a case where the water retention sheet 121C is formed on the steam generation layer 121A and the base material layer 121B is not provided, since there is a possibility that the steam generating portion 121 is in direct contact with the second sheet 122B, the second sheet 122B is preferably a sheet having water resistance.

In addition, the amount of steam generation per unit area of the steam generator 120 of the present embodiment is preferably equal to or more than 1 mg/cm$^2$ · 10 min, more preferably equal to or more than 1.5 mg/cm$^2$ · 10 min, still more preferably equal to or more than 5 mg/cm$^2$ · 10 min, still more preferably equal to or more than 7 mg/cm$^2$ • 10 min, and even more preferably equal to or more than 9 mg/cm$^2$ • 10 min, as the whole steam generator 120 from the viewpoint of imparting the user a moderate feeling of steam.

In addition, the amount of steam generation per unit area of the steam generator 120 of the present embodiment is preferably equal to or less than 20 mg/cm$^2$ · 10 min, more preferably equal to or less than 18 mg/cm$^2$ · 10 min, and still more preferably equal to or less than 15 mg/cm$^2$ · 10 min, as the whole steam generator 120 from the viewpoint of preventing dew condensation in the mask.

In addition, the amount of steam generation per unit area of the steam generator 120 of the present embodiment is preferably equal to or more than 1 mg/cm$^2$ · 10 min and equal to or less than 20 mg/cm$^2$ • 10 min, more preferably equal to or more than 1.5 mg/cm$^2$ · 10 min and equal to or less than 18 mg/cm$^2$ · 10 min, still more preferably equal to or more than 5 mg/cm$^2$ · 10 min and equal to or less than 15 mg/cm$^2$ · 10 min, still more preferably equal to or more than 7 mg/cm$^2$ · 10 min and equal to or less than 15 mg/cm$^2$ · 10 min, and even more preferably equal to or more than 9 mg/cm$^2$ · 10 min and equal to or less than 15 mg/cm$^2$ · 10 min, as the whole steam generator 120.

Next, the effect of the steam heating mask 100 will be described.

The steam heating mask 100 is provided with the mask 110 having the mask body portion 101 covering the mouth and the nose of the user, and the steam generator 120 held on a surface of the mask body portion 101 on a side of the user in which a space interposed between the mask body portion 101 and a face of the user is formed at the time of use, and the steam heating tool satisfies following formulae (1) to (3) .

$$300 \leq VG \leq 1,500 \qquad (1)$$

$$40 \leq MV \leq 150 \quad (2)$$

$$5 \leq VG/MV \leq 25 \quad (3)$$

In the formulae (1) to (3), VG represents a cumulative steam generation amount [mg] released into the space for 10 minutes from the start of steam generation of the steam generator 120, and MV represents a volume [mL] of the space.

According to the steam heating mask 100, by satisfying the formulae (1) to (3), steam can be effectively supplied to the user, so that feeling of steam and feeling of relaxation are obtained, and feeling of dryness of the nose and throat can be remedied. In addition, by satisfying the formula (2), the space covering the nose and mouth of the user has a moderate size, and feeling of wearing at the time of use is improved.

In addition, the steam heating mask 100 preferably satisfies the following formula (4).

$$30.0 \leq T \leq 45.0 \quad (4)$$

In the formula (4) T represents a maximum temperature [°C] of a nose portion of the user for 10 minutes from the start of steam generation of the steam generator 120.

In this manner, a moderate warm feeling can be obtained and the relaxation effect by steam can be further improved. That is, comfort due to heating and moistening and feeling of moist of mucous membranes of throat and nose are improved, breathing is eased by reducing discomfort due to nasal congestion, and a transport function of mucosal pilus is enhanced, so that an effect of promoting a discharge function of foreign substances is obtained. In this manner, an effect of cold prevention can be expected. Furthermore, sleepy feeling is also induced. In addition, since a lip portion is sensitive to temperature because a stratum corneum is thin, as the steam generator 120 warms a lateral portion of the nose, it is possible to reduce the feeling that the lip portion is likely to feel excessively hot.

In addition, the temperature of the nose portion of the user can be measured as follows.

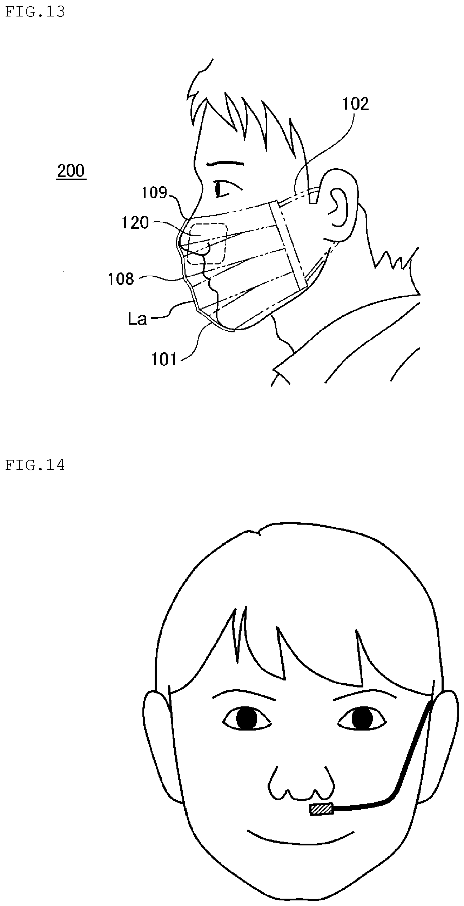

As shown in Fig. 14, before wearing the steam heating mask 100, a digital temperature and humidity sensor SH-T75 (manufactured by Sensirion Co., Ltd.) is disposed in front of the hole of the nose so as not to touch the skin, and fixed with a surgical tape so as not to move. Under a 20°C and 60% RH environment, the steam heating mask is worn and change in temperature and humidity are measured every second by using the temperature and humidity evaluation kit EK-H4 (manufactured by Sensirion Co., Ltd.).

The steam heating mask 100 is used, for example, as follows.

That is, when the mask 110 and the steam generator 120 are separated, the steam heating mask 100 opens the oxygen blocking bag, takes out the steam generator 120, and fixes it at a predetermined position of the mask 110. The steam heating mask 100 is worn so that the mask body portion 101 covers the mouth and the nose of the user by hanging each ear hooking portion 102 on the ear of the user.

In addition, in a case where the steam generator 120 is preliminarily sealed in the housing body 104 of the mask 110, the steam generator 120 is normally sealed in the oxygen blocking bag as a whole of the steam heating mask 100. As a method of use in this case, after opening the oxygen blocking bag, the steam heating mask 100 is taken out, and is worn so that the mask body portion 101 covers the mouth and nose of the user by hanging each ear hooking portion 102 on the ear of the user.

The mask body portion 101 holding the steam generator 120 is used so as to cover the mouth and nose of the user, so that the steam generated in the mask body portion 101 by the steam generator 120 is inhaled from the mouth and nose of the user.

(Second Embodiment)

In the first embodiment, the steam heating mask 100 in which the mask body portion 101 has an arc-like folding line 103 on the vertical center portion and a space is formed by being unfolded from a state folded in half by the folding line 103 is described, and in the second embodiment, a steam heating mask 200 in which the mask body portion 101 has a pleat portion in the lateral direction will be described. Hereinafter, description of configurations and effects similar to those of the first embodiment may not be repeated.

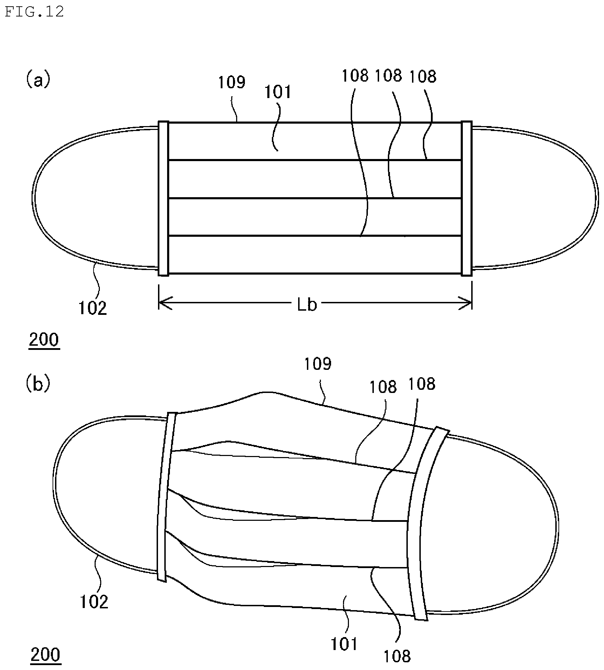

Fig. 12(a) is a perspective view schematically showing a state before use of the steam heating mask 200 according to the second embodiment. Fig. 12 (b) is a perspective view schematically showing a state of use of the steam heating mask 200 according to the second embodiment. Fig. 13 is a cross-sectional view of the steam heating mask 200 according to the second embodiment in the state of use.

As shown in Fig. 12(a), the steam heating mask 200 has three pleat portions 108 in the lateral direction (longitudinal direction) of the mask body portion 101. In this manner, the mask body portion 101 is foldable. As shown in Fig. 12(b), at the time of use, the pleat portion 108 is widened in the vertical direction (short direction) of the mask body portion 101 from the folded state and a space covering the nose and mouth of the user can be formed. That is, by being used in a state unfolded from the folded state, a space interposed between the mask body portion 101 and the face of the user can be formed.

The number of pleat portions 108 may be one, two, or may be equal to or more than four.

In the second embodiment, as shown in Fig. 13, MV is calculated as a volume of the space interposed between the mask body portion 101 and the face of the user, when the pleat portion 108 of the mask body portion 101 is unfolded and the steam heating mask 200 is worn.

In addition, in the second embodiment, as shown in Fig. 13, a maximum length (La) of the vertical center line of the mask body portion 101 is a length (path) from an uppermost end to a lowermost end of the mask body portion 101, when the pleat portion 108 of the mask body portion 101 is unfolded and the steam heating mask 200 is properly worn.

In the second embodiment, the maximum length (La) of the vertical center line of the mask body portion 101 is preferably 12 to 20 cm. In this manner, a space of an appropriate size is formed between the mask body portion 101 and the face of the user.

In addition, a length (Lb) of the upper side of the mask body portion 101 is shown in Fig. 12 (a) . In the second embodiment, a ratio of the maximum length (La) of the vertical center line of the mask body portion 101 to a sum of the length (Lb) of the upper side of the mask body portion 101 is preferably 0.6 to 1.4.

In this manner, the mask 110 can come into close contact with the face of the user, and the space of an appropriate size is formed between the mask body portion 101 and the face of the user.

The steam heating mask 200 is provided with a flexible member 109 on the upper side of the mask body portion 101. In this manner, the shape of the mask body portion 101 can be deformed to conform to the unevenness of the face of the user, and the deformed shape can be maintained. Therefore, the mask body portion 101 and the face of the user can be more closely contacted with each other. Examples of the flexible member 109 include a wire made of resin or metal.

In the first embodiment and the second embodiment, although an example in which the ear hooking portion 102 is provided has been described, the ear hooking portion 102 may not be provided, and the mask body portion 101 may be held by the user with a hand or the like and brought into close contact with the face of the user. In this case, the shape of the mask body portion 101 is not particularly limited, and the shape may be a cup shape or a dome shape. Alternatively, the mask body portion 101 having the cup shape or the dome shape may be accommodated in the mask having the normal ear hooking portion, and the mask body portion 101 may be brought into close contact so as to cover the nose and mouth of the user.

In addition, in the first embodiment and the second embodiment, although an example in which the ear hooking portion 102 is provided as a fixing portion fixing the steam heating tool 100 to the face of the user is described, as the fixing portion fixing the steam heating tool 100 to the face of the user, the fixing portion may be fixed to the face of the user by wearing the fixing portion on the head portion. For example, a stretchable band, a pair of bands, or the like may be worn on the head portion.

(Third Embodiment)

In the first embodiment, the steam heating mask 100 in which the housing body 104 is used as the fixing means is described. In the third embodiment, a steam heating mask 300 in which an adhesive is used as the fixing means will be described. Hereinafter, description of configurations and effects similar to those of the first embodiment and the second embodiment may not be repeated.

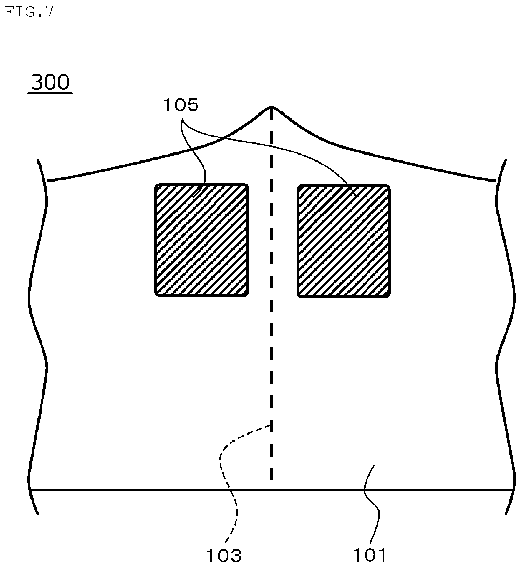

Fig. 7 is a partial plan view when seen from the surface on the side of the user before wearing the steam generator to the steam heating mask according to the third embodiment. Fig. 8 is a cross-sectional view of a portion of the mask according to the third embodiment when seen from an upper surface (eye side of user).

[Mask]

Materials similar to those in the first embodiment and the second embodiment can be used for the mask body portion 101.

In the present embodiment, as shown in Fig. 7, it is preferable that a marking region 105 is formed on the surface of the mask body portion 101 on the side of the user of the steam heating mask 300 so that the position where the steam generator 120 is attached can be known. As shown in Fig. 8, the steam generator 120 is fixed to the mask body portion 101 on the marking region 105 through an adhesive 106. In the marking region 105, the color in the region may be changed by printing or emboss processing may be performed. In addition, the marking region 105 may be provided with lines such as a solid line and a dotted line around the marking region 105.

In addition, from the viewpoint of improving the feeling of wearing, a nonwoven fabric such as an air-through nonwoven fabric which is a sheet material having good texture may be disposed between the first sheet 122A of the steam generator 120 and the user (not shown). In this case, the nonwoven fabric preferably has air permeability so as not to inhibit passage of steam. Furthermore, the nonwoven fabric more preferably has water repellency so as not to inhibit the passage of steam and not to inhibit the inflow of air, due to the wetting by steam.

Such a nonwoven fabric may be formed on the first sheet 122A of the steam generator 120, and may be attached to the surface of the mask 110 on the side of the user so as to be freely opened and closed and closed after attaching the steam generator 120.

[Adhesive]

In the present embodiment, the adhesive 106 is provided on a surface of the steam generator 120 opposite to the side of the user, that is, a surface of the second sheet 122B opposite to the side of the user. In this manner, the steam generator 120 can be stably fixed to the mask 110.

The size and shape of the adhesive 106 are not particularly limited as long as the adhesive 106 can fix at least the steam generator 120 to the mask body portion 101.

As the adhesive 106, a hot melt adhesive is preferably used. The hot melt adhesive normally contains an adhesive base, a tackifier resin and a softening agent as constituent components. Examples of the hot melt adhesive include synthetic rubber type, polyolefin type (polyethylene (PE) type, ethylene vinyl acetate (EVA) type, ethylene-ethyl-acrylate (EEA) type, atactic polypropylene (APP) type, amorphous poly alpha olefin (APAO) type, and the like), polyamide type (nylon type, polyamide type, and the like), polyester type, acrylic type, and the like. These adhesives can be used alone or in combination of equal to or more than two types . From the viewpoints of preservability, adhesive strength, safety, and the like, synthetic rubber type, polyolefin type, acrylic type, and polyamide type are preferable, and synthetic rubber type is particularly preferable.

The adhesive 106 is protected by release paper in a state before use of the steam heating mask 300 and does not adhere to the outside. The release paper is not particularly limited and any release paper can be used.

The adhesive may be provided on the surface of the mask body portion 101 on the side of the user. Specifically, the adhesives are provided in pairs symmetrically in the vicinity of the vertical center line of the mask body portion 101 and in the vicinity of the upper end portion of the mask body portion 101. In this manner, the fixing position of the steam generator 120 can be easily understood. In this case, it is preferable to use the adhesive that can be used repeatedly.

Hereinbefore, although the embodiments of the present invention have been described with reference to the drawings, these are examples of the present invention, and various configurations other than the above can be adopted within the scope of appended claims.

In the first embodiment, although an example in which the degree of air permeability of the first sheet is equal to or less than 7, 000 sec/100 ml and the degree of air permeability of the second sheet is more than 8,000 sec/100 ml has been described, the degree of air permeability of the second sheet is equal to or more than 250 sec/100 ml and equal to or less than 8,000 sec/100 ml and the degree of air permeability of the first sheet may be equal to or less than 20% of the degree of air permeability of the second sheet. In this manner, steam generated by the steam generating portion 121 can be prevented from leaking to the outside of the mask 110, and the steam can be applied inside the mask 110, that is, to the side of the user. In this manner, warmed steam is positively generated in the mask, and the absolute humidity can be increased in the mask, so that comfort due to heating and moistening and feeling of moist of mucous membranes of throat and nose can be improved. In addition, with such an effect, breathing is eased by reducing discomfort due to nasal congestion, and a transport function of mucosal pilus is enhanced, so that an effect of promoting a discharge function of foreign substances is obtained.

Although the second sheet 122B may be an air permeable sheet of which a portion has the air permeability, or may be an air impermeable sheet of which a portion does not have the air permeability, and the degree of air permeability of the second sheet 122B is preferably equal to or more than 250 sec/100 ml and equal to or less than 8,000 sec/100 ml as a whole. In addition, from the viewpoints of preventing abnormal heat generation of the steam generator, appropriately distributing the steam generated from the steam generating portion 121 and sufficiently applying the steam to side of the user, the degree of air permeability of the second sheet 122B is more preferably equal to or more than 4,000 sec/100 ml and equal to or less than 7,500 sec/100 ml, and still more preferably equal to or more than 5,000 sec/100 ml and equal to or less than 7,000 sec/100 ml.

From the viewpoint of appropriately distributing the steam generated from the steam generating portion 121 and sufficiently applying the steam to the side of the user, and the viewpoint of preventing the swelling of the steam generator and preventing stuffiness when wearing the steam heating mask, the degree of air permeability of the first sheet 122A is preferably equal to or less than 20%, more preferably equal to or less than 10%, and further more preferably in the order of equal to or less than 5%, equal to or less than 3%, equal to or less than 1%, and equal to or less than 0% of the degree of air permeability of the second sheet 122B.

Although the degree of air permeability of the first sheet 122A can be appropriately selected from those satisfying the condition that the degree of air permeability of the first sheet 122A is equal to or less than 20% of the degree of air permeability of the second sheet 122B, and specifically, the degree of air permeability of the first sheet 122A is preferably equal to or less than 1,600 sec/100 ml, more preferably equal to or less than 1,000 sec/100 ml, and further more preferably in the order of equal to or less than 250 sec/100 ml, equal to or less than 10 sec/100 ml, equal to or less than 5 sec/100 ml, and equal to or less than 0 sec/100 ml, from the viewpoint of appropriately distributing the steam generated from the steam generating portion 121 and sufficiently applying the steam to the side of the user, and the viewpoint of preventing the swelling of the steam generator and preventing stuffiness when wearing the steam heating mask.

As the first sheet 122A and the second sheet 122B, a sheet using the same material as that of the sheet described in the first embodiment can be used as long as the above degree of air permeability is satisfied.

In addition, in the above embodiment, although the example in which two steam generators 120 are separately attached to the mask 110 has been described, it may have a structure in which two steam generators 120 are connected by one bag.

In addition, in the above embodiment, although the example in which the mask body portion 101 is formed of one sheet and folded symmetrically with the folding line 103 has been described, the mask body portion 101 may be one in which two sheets of the same shape are overlapped and a folding line 103 is formed by sticking one side. As the sheet used in this case, the same sheet as described in the above embodiment can be used.

Hereinbefore, although the embodiments of the present invention have been described with reference to the drawings, these are examples of the present invention, and subject to the appended claims, various configurations other than the above can be adopted within the scope of appended claims.

[Example]

Next, although the present invention will be described in detail by examples, the content of the present invention is not limited to the examples but only by the appended claims.

<Preparation of Steam Generating Portion>

A heating composition having the composition shown in Table 1 was prepared by the following procedure.

The thickener was dissolved in water, then tripotassium phosphate was dissolved to prepare an aqueous solution. On the other hand, a powder premixed with iron powder and activated carbon was prepared, and the premixed powder was placed in the aqueous solution and stirred with a disk turbine type stirring blade at 150 rpm for 10 minutes to obtain a slurry-like heating composition.

Next, under the conditions shown in Table 2, a steam generation layer was formed to prepare steam generating portions A to F. In Table 2, the coating amount, the amount of salt, and the spraying amount of the water absorbing polymer were shown as the amount (g/24 cm$^2$) per one (4.9 cm $\times$ 4.9 cm; area 24.0 cm$^2$) of the steam generating portion.

Specifically, using the die coating method, the obtained heating composition was coated to one side of the base material layer. As the base material layer, polyethylene laminated paper (manufactured by Nittoku Co., Ltd.) was used. Next, salt (sodium chloride designated by Japanese Pharmacopoeia (manufactured by Otsuka Pharmaceutical Co., Ltd.)) was sprayed on the coated surface to form a steam generation layer. Subsequently, the water absorbing polymer (sodium polyacrylate, spherical, average particle size 300 pm, Aqualic CA, manufactured by Nippon Shokubai Co., Ltd.) was further sprayed directly onto the side of the obtained steam generation layer to which the heating composition was coated.

Subsequently, crepe papers as water absorbing sheets (manufactured by Daishowa Paper Industries Co., Ltd.) were laminated on the above sprayed water absorbing polymer and integrated to prepare steam generating portions A to C, respectively.

In addition, a water retention sheet was overlapped on the obtained steam generation layer to prepare a steam generating portion D. As the water retention sheet, a polymer sheet in which a wood pulp paper (basis weight 20 g/m$^2$, manufactured by Ino Paper Co., Ltd.), a water absorbing polymer (sodium polyacrylate, spherical, average particle size 300 pm, basis weight 70 g/m$^2$, Aqualic CA, manufactured by Nippon Shokubai Co., Ltd.), and a wood pulp paper (basis weight 30 g/m$^2$, manufactured by Ino Paper Co., Ltd.) were laminated and integrated was used.

In addition, the obtained steam generating portion D was overlapped on two layers such that the water retention sheet was disposed on the skin side (first sheet side) to prepare a steam generating portion E.

In addition, the obtained steam generating portion D was overlapped on three layers such that the water retention sheet was disposed on the skin side (first sheet side) to prepare a steam generating portion F.

[Table 1]

[Table 1]

| Component | | Parts by mass |
|---|---|---|
| Iron powder | Iron powder RKH: Manufactured by Dowa Electronics Co., Ltd. | 100 |
| Activated carbon | Carborafin: Manufactured by Japan EnviroChemicals Co., Ltd. | 8 |
| Thickener | Xanthan gum: DSP Gokyo Food ε Chemical Co., Ltd. | 0.25 |
| pH adjusting agent | Ttripotassium phosphate: Yoneyama Chemical Industry Co., Ltd. | 1.8 |
| Water | Ion exchanged water | 62 |
| | Total | 172.05 |

[Table 2]

[Table 2]

|  |  |  |  | A | B | C | D | E | F |
|---|---|---|---|---|---|---|---|---|---|
| Steam generating portion | Steam generation layer | Coating amount | g/24 cm$^2$ | 1.4 | 3.0 | 3.2 | 4.6 | Two pieces of D are overlapped | Three pieces of D are overlapped |
|  |  | Amount of salt | g/24 cm$^2$ | 0.07 | 0.15 | 0.16 | 0.23 |  |  |
|  | Water retention layer | Spraying amount of water absorbing polymer | g/24 cm$^2$ | 0.06 | 0.12 | 0.12 |  |  |  |
| Steam generator | First sheet | | | TSF500*1 | Syntex SMS*2 | Syntex SMS | Syntex SMS | Syntex SMS | Syntex SMS |
|  | Second sheet | | | LF-KIPE71*3 | LF-KIPE71 | LF-KIPE71 | LF-KIPE71 | LF-KIPE71 | LF-KIPE71 |
| *1: "TSF500" manufactured by Kohjin Co., Ltd., degree of air permeability 500 sec/100 ml<br>*2: "Syntex SMS" manufactured by Mitsui Chemicals, Inc., degree of air permeability 0 sec/100 ml<br>*3: "LF-KIPE71" manufactured by Kakukei Co., Ltd., air impermeable, basis weight 71 g/m$^2$ | | | | | | | | | |

<Preparation of Steam Generator>

Each of the obtained steam generating portions A to F was covered with a bag formed of a first sheet and a second sheet to prepare a steam generator.

Specifically, the first sheet and the second sheet shown in Table 2 were prepared. Each of the steam generating portions A to F was disposed between the first sheet and the second sheet and the peripheral portions were tightly sealed to obtain steam generators. At this time, the base material layers of the steam generating portions A to F were disposed on the second sheet side. In this case, an area of the first sheet including an air permeable surface and a seal portion was 39.7 cm$^2$ (6.3 cm $\times$ 6.3 cm) . This was used as one piece.

The steam generator was stored in an oxygen blocking bag until evaluation described later was performed.

<Preparation of Steam Heating Mask>

As the sheet of the mask body portion to be a portion covering the mouth and nose, two layers were overlapped so that the SMS nonwoven fabrics (spunbond (polypropylene)-meltblown (polypropylene)-spunbond (polypropylene) laminated integrated type, basis weight 50 g/m$^2$) were on the outside and the spunbonded nonwoven fabric (polypropylene) with a basis weight of 25 g were on the inside (on user side) . At this time, a blocking ratio of particles of equal to or more than 0.3 $\mu$m in the mask body portion was 25%. On the upper portion of both sides across the vertical center folding line of the mask body portion, one by one (two in total) housing body containing the steam generator was prepared. Each housing body was prepared by thermally fusing portions of the housing body other than the upper portion of the mask. A pair of stretchable rubber string-like ear hooking portions were attached to the end portion of the mask body portion to prepare a mask having a three-dimensional shape.

When the air permeation resistance of the mask body portion was measured with the housing body portion, the air permeation resistance was 148 Pa/30 mm$\Phi$ · pressure difference. In addition, one by one of the steam generators (two in total) is placed in the housing body from the upper side of the mask to which two nonwoven fabrics of the mask body portion were not thermally fused, and a pair of the above steam generators were attached symmetrically to the vicinity of the folding line of the mask body portion and the vicinity of the upper end portion (nasal side) of the mask body portion to form a steam heating mask.

The protrusion amount of the mask body portion at the time of wearing the steam heating mask was changed, without changing the area of the region covering the mouth and nose to prepare the steam heating masks having five different MV values.

The MV value represents the volume [mL] of the space interposed between the mask body portion and the face of the user at the time of using the steam heating mask.

. Examples and Comparative Examples

Using the obtained steam heating masks, the following measurements and evaluations were performed. The results are shown in Table 2.

<Measurement of Amount of Steam Generation>

The amount of steam generation was measured as follows using a device 30 shown in Fig. 11.

The device 30 shown in Fig. 11 includes an aluminum measurement chamber (volume 4.2 L) 31, an inflow path 32 through which dehumidified air (humidity less than 2%, flow rate 2.1 L/min) flows into the lower portion of the measurement chamber 31, an outflow path 33 through which air flows out from the upper portion of the measurement chamber 31, an inlet temperature and humidity meter 34 and an inlet flow meter 35 provided in the inflow path 32, an outlet temperature and humidity meter 36 and an outlet flow meter 37 provided in the outflow path 33, and a thermometer (thermistor) 38 provided in the measurement chamber 31. As the thermometer 38, a thermometer having a temperature resolution of approximately 0.01°C was used.

Measurement of the surface temperature of a surface located on the skin side of the steam generator 120 was performed by taking out the steam generator 120 from the oxygen blocking bag at the temperature 30°C (30°C $\pm$ 1°C) of the measurement environment, placing the surface located on the skin side of the steam generator 120 or the surface located on the skin side of the steam heating mask 100 facing upward, that is, placing a surface releasing the steam facing upward on the measurement chamber 31, and placing the thermometer 38 with a metal ball (4.5 g) on the above-mentioned steam release side. In addition, in this state, the dehumidified air flowed from the lower portion, and the difference of the absolute humidity between before and after the air flows into the measurement chamber 31 was obtained from the temperature and the humidity measured by the inlet temperature and humidity meter 34 and the outlet temperature and humidity meter 36. Furthermore, the amount of steam released by the steam generator 120 or the steam heating mask 100 was calculated from the flow rate measured by the inlet flow meter 35 and the outlet flow meter 37.

The amount of steam generation refers to a total amount of steam measured from the time when the steam generator 120 is removed from the oxygen blocking bag as the starting point and until 10 minutes later.

<Measurement of MV Value>

The volume of the space interposed between the mask body portion and a face of a subject was measured as follows. Three-dimensional shape data of each of the face of the subject and the face to which the mask was worn was acquired with a three-dimensional measuring instrument DANAE 100 (manufactured by NEC Corporation). The two three-dimensional shape data obtained were overlapped by three-dimensional image analysis software 3D-RUGLE (manufactured by Medic Engineering Co., Ltd.), the volume of the protrusion portion was calculated by wearing the mask. The volume occupied by the mask body was subtracted from this value, and was set as the MV value (mL) . The obtained MV value was substantially the same as the method of disposing a liquid impermeable film inside the mask, pouring water into this portion, putting the subject's face to a downward direction so that the subject is in the same state as the mask was worn, and measuring the amount of water remaining in the mask after the face was taken off from the mask, as disclosed in Japanese Unexamined Patent Publication No. 2012-205926. In addition, the obtained MV value was substantially the same as an MV value measured using the human head model modeled using the average human head data manufactured by Digital Human Technology Co., Ltd.

<Measurement of Maximum Length (La) of Vertical Center Line of Mask Body Portion and Length (Lb) of Upper Side of Mask Body Portion>

The maximum length (La) of the vertical center line of the mask body portion and the length (Lb) of the upper side of the mask body portion were measured using a tape measure with the mask worn on the face.

<Measurement of Temperature ($T_{max}$)>

As shown in Fig. 14, the digital temperature and humidity sensor SH-T75 (manufactured by Sensirion Co., Ltd.) was disposed in front of the hole of the nose of the user so as not to touch the skin and was fixed to the face with a surgical tape so as to keep it immobilized, before wearing the steam heating mask. Under the environment of 20°C and 60% RH, the steam heating mask was worn, and the temperature and humidity change per second was measured with the temperature and humidity evaluation kit EK-H4 (manufactured by Sensirion Co., Ltd.). The maximum temperature of the temperature measured in 10 minutes from the start of steam generation of the steam generator was set as $T_{max}$ [°C].

<Evaluation>

Under the environment of 22°C, feeling of wearing of the steam heating mask was evaluated by five male subjects. Feeling of warmth, feeling of steam (moisture), a change in sensation, and a change in mood with the steam heating mask worn are evaluated according to the following criteria, and the scores with the most number of people were selected and scored.

· Feeling of wearing

1: Small and stuffy feeling
2: Slightly small
3: Just a good size
4: Slightly large
5: Large and disturbing feeling

· Feeling of warmth

1: Tepid
2: Slightly tepid
3: Just a good warmth
4: Slightly hot
5: Hot

· Feeling of steam

1: Amount of steam is low
2: Amount of steam is slightly low
3: Just a good amount of steam
4: Amount of steam is slightly high
5: Amount of steam is high

· Change in sensation (30 minutes after wearing steam heating mask)

1: Feeling of dryness of nose and throat did not improve at all
2: Feeling of dryness of nose and throat was slightly improved
3: Feeling of dryness of nose and throat was improved a little
4: Feeling of dryness of nose and throat was improved
5: Feeling of dryness of nose and throat was remarkably improved

· Change in mood (30 minutes after wearing steam heating mask)

1: Nothing changed
2: Mood slightly relaxed
3: Mood relaxed a little
4: Good mood relaxed
5: Good mood significantly relaxed

[Table 3]

| | Example | | | | | | | | | | Comparative Example | | | | | | | | |
|---|---|---|---|---|---|---|---|---|---|---|---|---|---|---|---|---|---|---|---|
| | 1 | 2 | 3 | 4 | 5 | 6 | 7 | 8 | 9 | 10 | 1 | 2 | 3 | 4 | 5 | 6 | 7 | 8 | 9 |
| Types of steam generating portion | D | C | B | E | C | E | D | C | E | D | C | A | E | A | F | B | F | E | C |
| (a) Cumulative steam generation amount for 10 minutes (mg) | 547 | 333 | 243 | 650 | 333 | 650 | 547 | 333 | 650 | 547 | 333 | 128 | 650 | 128 | 800 | 243 | 800 | 650 | 333 |
| (b) Number of steam generators used (pieces) | 2 | 2 | 2 | 2 | 2 | 2 | 2 | 2 | 2 | 2 | 2 | 2 | 2 | 2 | 2 | 2 | 2 | 2 | 2 |
| VG(mg/10min):(a)×(b) | 1094 | 666 | 486 | 1300 | 666 | 1300 | 1094 | 666 | 1300 | 1094 | 666 | 256 | 1300 | 256 | 1600 | 486 | 1600 | 1300 | 666 |
| MV(mL) | 50.1 | 50.1 | 50.1 | 70 | 70 | 99.5 | 99.5 | 99.5 | 128.7 | 128.7 | 30.5 | 30.5 | 50.1 | 50.1 | 99.5 | 99.5 | 128.7 | 160 | 160 |
| VG/MV | 21.8 | 13.3 | 9.7 | 18.6 | 9.5 | 13.1 | 11.0 | 6.7 | 10.1 | 8.5 | 21.8 | 8.4 | 25.9 | 5.1 | 16.1 | 4.9 | 12.4 | 8.1 | 4.2 |
| La(cm) | 15.0 | 15.0 | 15.0 | 15.7 | 15.7 | 16.5 | 16.5 | 16.5 | 17.5 | 17.5 | 14.0 | 14.0 | 15.0 | 15.0 | 16.5 | 16.5 | 17.5 | 18.5 | 18.5 |
| La/Ib | 1.0 | 1.0 | 1.0 | 1.1 | 1.1 | 1.1 | 1.1 | 1.1 | 1.2 | 1.2 | 0.9 | 0.9 | 1.0 | 1.0 | 1.1 | 1.1 | 1.2 | 1.2 | 1.2 |
| Tmax(°C) | 41.1 | 36.3 | 30.3 | 44.9 | 34.5 | 44.8 | 37.9 | 32.0 | 32.9 | 31.6 | 35.5 | 30.2 | 51.2 | 27.1 | 47.4 | 30.1 | 46.6 | 33.5 | 29.2 |
| Evaluation — Feeling of wearing | 2 | 2 | 2 | 3 | 3 | 4 | 4 | 4 | 4 | 4 | 1 | 1 | 2 | 2 | 4 | 4 | 4 | 5 | 5 |
| Evaluation — Feeling of warmth | 4 | 3 | 2 | 4 | 2 | 4 | 3 | 2 | 3 | 2 | 5 | 1 | 5 | 2 | 5 | 1 | 5 | 2 | 1 |
| Evaluation — Feeling of steam | 4 | 3 | 2 | 4 | 2 | 3 | 3 | 2 | 3 | 2 | 5 | 1 | 5 | 2 | 5 | 1 | 5 | 2 | 1 |
| Evaluation — Change in sensation | 4 | 5 | 4 | 4 | 5 | 5 | 5 | 4 | 5 | 4 | 4 | 1 | 4 | 1 | 3 | 1 | 3 | 3 | 1 |
| Evaluation — Change in mood | 4 | 5 | 4 | 5 | 4 | 5 | 5 | 4 | 5 | 4 | 2 | 1 | 2 | 2 | 2 | 1 | 2 | 2 | 1 |

**Claims**

1. A steam heating tool (100) comprising:

   a cover member (101) that has a cover portion covering a mouth and a nose of a user; and
   a steam generator (120) that is held on a surface of the cover portion on a side of the user,
   wherein a space interposed between the cover portion and a face of the user is formed at a time of use, and
   **characterized in that**
   the steam heating tool satisfies following formulae (1) to (3).

$$300 \leq VG \leq 1,500 \qquad (1)$$

$$40 \leq MV \leq 150 \qquad (2)$$

$$5 \leq VG/MV \leq 25 \qquad (3)$$

   In the formulae (1) to (3), VG represents a cumulative steam generation amount [mg] released into the space for 10 minutes from a start of steam generation of the steam generator, and MV represents a volume [mL] of the space.

2. The steam heating tool according to claim 1,
   wherein a maximum length (La) of a vertical center line of the cover portion of the steam heating tool is 12 to 20 cm.

3. The steam heating tool according to claim 1 or 2,
   wherein a ratio (La/Lb) of the maximum length (La) of the vertical center line of the cover portion to a length (Lb) of an upper side of the cover portion is 0.6 to 1.7.

4. The steam heating tool according to any one of claims 1 to 3, which satisfies the following formula (4).

$$30.0 \leq T \leq 45.0 \qquad (4)$$

   In the formula (4), T represents a maximum temperature [°C] of a nose portion of the user for 10 minutes from the start of steam generation of the steam generator.

5. The steam heating tool according to any one of claims 1 to 4,
   wherein the steam generator is held in a region that covers the nose of the user.

6. The steam heating tool according to any one of claims 1 to 5,
   wherein the cover member has a fixing portion (102) that fixes the steam heating tool to the face of the user.

7. The steam heating tool according to any one of claims 1 to 6,
   wherein the cover portion is configured to be foldable and used in an unfolded state from a folded state to form the space.

8. The steam heating tool according to claim 7,
   wherein the cover portion has a folding line (103) on the vertical center portion and is unfolded from a state folded in half by the folding line to form the space.

9. The steam heating tool according to any one of claims 1 to 8,
   wherein the steam generator generates steam by an oxidation reaction of an oxidizable metal.

**Patentansprüche**

1. Dampfheizgerät (100), umfassend:

   ein Abdeckungselement (101), das einen Abdeckungsbereich aufweist, der einen Mund und eine Nase eines Benutzers abdeckt; und
   einen Dampferzeuger (120), der auf einer Oberfläche des Abdeckungsbereich auf einer Seite des Benutzers gehalten wird,
   wobei ein Raum zwischen dem Abdeckungsbereich und einem Gesicht des Benutzers zum Zeitpunkt der Verwendung gebildet wird, und
   **dadurch gekennzeichnet, dass** das Dampfheizgerät die folgenden Formeln (1) bis (3) erfüllt:

$$300 \leq VG \leq 1.500 \quad (1)$$

$$40 \leq MV \leq 150 \quad (2)$$

$$5 \leq VG/MV \leq 25 \quad (3)$$

   wobei in den Formeln (1) bis (3) VG für eine kumulative Dampferzeugungsmenge [mg] steht, die innerhalb von 10 Minuten ab Beginn der Dampferzeugung des Dampferzeugers in den Raum abgegeben wird, und MV für ein Volumen [ml] des Raums steht.

2. Dampfheizgerät gemäß Anspruch 1,
   wobei eine maximale Länge (La) einer vertikalen Mittellinie des Abdeckungsbereichs des Dampfheizgeräts 12 bis 20 cm beträgt.

3. Dampfheizgerät gemäß Anspruch 1 oder 2,
   wobei ein Verhältnis (La/Lb) von der maximalen Länge (La) der vertikalen Mittellinie des Abdeckungsbereichs zu einer Länge (Lb) einer Oberseite des Abdeckungsbereichs 0,6 bis 1,7 beträgt.

4. Dampfheizgerät gemäß einem der Ansprüche 1 bis 3, das die folgende Formel (4) erfüllt:

$$30,0 \leq T \leq 45,0 \quad (4)$$

   wobei in der Formel (4) T für eine maximale Temperatur [°C] eines Nasenbereichs des Benutzers innerhalb von 10 Minuten nach Beginn der Dampferzeugung durch den Dampferzeuger steht.

5. Dampfheizgerät gemäß einem der Ansprüche 1 bis 4,
   wobei der Dampferzeuger in einem Bereich gehalten wird, der die Nase des Benutzers bedeckt.

6. Dampfheizgerät gemäß einem der Ansprüche 1 bis 5,
   wobei das Abdeckelement einen Fixierabschnitt (102) aufweist, der das Dampfheizgerät am Gesicht des Benutzers fixiert.

7. Dampfheizgerät gemäß einem der Ansprüche 1 bis 6,
   wobei der Abdeckungsbereich so konfiguriert ist, dass er faltbar ist und in einem entfalteten Zustand aus einem gefalteten Zustand verwendet wird, um den Raum zu bilden.

8. Dampfheizgerät gemäß Anspruch 7,
   wobei der Abdeckungsbereich eine Faltlinie (103) auf dem vertikalen Mittelabschnitt aufweist und aus einem durch die Faltlinie in die Hälfte gefalteten Zustand entfaltet wird, um den Raum zu bilden.

9. Dampfheizgerät gemäß einem der Ansprüche 1 bis 8,
   wobei der Dampferzeuger Dampf durch eine Oxidationsreaktion eines oxidierbaren Metalls erzeugt.

**Revendications**

1.  Outil de chauffage à vapeur (100) comprenant :

    un élément de couvercle (101) qui présente une partie de couvercle couvrant la bouche et le nez d'un utilisateur ; et
    un générateur de vapeur (120) qui est maintenu sur une surface de la partie de couvercle sur un côté de l'utilisateur,
    dans lequel un espace interposé entre la partie de couvercle et le visage de l'utilisateur est formé lors d'un moment d'utilisation, et
    **caractérisé en ce que**
    l'outil de chauffage à vapeur satisfait aux formules suivantes (1) à (3).

    $$300 \leq VG \leq 1\,500 \quad (1)$$

    $$40 \leq MV \leq 150 \quad (2)$$

    $$5 \leq VG/MV \leq 25 \quad (3)$$

    dans les formules (1) à (3), VG représente une quantité de génération de vapeur cumulée [mg] libérée dans l'espace pendant 10 minutes à partir d'un début de génération de vapeur par le générateur de vapeur, et MV représente un volume [mL] de l'espace.

2.  Outil de chauffage à vapeur selon la revendication 1,
    dans lequel une longueur maximale (La) d'une ligne centrale verticale de la partie de couvercle de l'outil de chauffage à vapeur est comprise de 12 à 20 cm.

3.  Outil de chauffage à vapeur selon la revendication 1 ou la revendication 2,
    dans lequel un rapport (La/Lb) de la longueur maximale (La) de la ligne centrale verticale de la partie de couvercle sur une longueur (Lb) d'un côté supérieur de la partie de couvercle est compris de 0,6 à 1,7.

4.  Outil de chauffage à vapeur selon l'une quelconque des revendications 1 à 3, qui satisfait à la formule suivante (4).

    $$30,0 \leq T \leq 45,0 \quad (4)$$

    dans la formule (4), T représente une température maximale [°C] d'une partie de nez de l'utilisateur pendant 10 minutes à partir du début de génération de vapeur par le générateur de vapeur.

5.  Outil de chauffage à vapeur selon l'une quelconque des revendications 1 à 4,
    dans lequel le générateur de vapeur est maintenu dans une région qui couvre le nez de l'utilisateur.

6.  Outil de chauffage à vapeur selon l'une quelconque des revendications 1 à 5,
    dans lequel l'élément de couvercle présente une partie de fixation (102) qui fixe l'outil de chauffage à vapeur au visage de l'utilisateur.

7.  Outil de chauffage à vapeur selon l'une quelconque des revendications 1 à 6,
    dans lequel la partie de couvercle est configurée pour pouvoir être pliée et utilisée dans un état déplié à partir d'un état plié pour former l'espace.

8.  Outil de chauffage à vapeur selon la revendication 7,
    dans lequel la partie de couvercle présente une ligne de pliage (103) sur la partie centrale verticale et est dépliée à partir d'un état plié en deux par la ligne de pliage pour former l'espace.

9.  Outil de chauffage à vapeur selon l'une quelconque des revendications 1 à 8,

dans lequel le générateur de vapeur génère de la vapeur par une réaction d'oxydation d'un métal oxydable.

FIG.1

FIG.2

100

102

120

101

FIG.3

100

120

104

107a

101

103

FIG.4

100

104　　　　　　120　　　　　　　　104

103

101

FIG.5

100a　　　　120　　　　　104

107b

103　　　　101

FIG.6

FIG.7

FIG.8

300

120 120

106 103 106 101

FIG.9

120

122A 121C 122

122B 121B 121A 121

FIG.10

FIG.11

FIG.12

(a)

200

(b)

200

FIG.13

FIG.14

**REFERENCES CITED IN THE DESCRIPTION**

**Patent documents cited in the description**

- JP 2004073828 A
- JP 2004358110 A
- JP 2004016753 A
- JP 2011136060 A
- JP 2012205926 A
- JP 2003102761 A
- JP 2013146554 A